# EUROPEAN PATENT APPLICATION

(11) **EP 4 751 734 A1**
(43) Date of publication of application: **03.06.2026**
(21) Application number: 24216627.0
(22) Date of filing: 29.11.2024
(51) Int. Cl.: A61K 39/395, A61P 35/00, C07K 16/28, A61K 39/00

(54) **BISPECIFIC ANTIBODY CONSTRUCT DOSAGE REGIMEN FOR TREATING NSCLC**

(71) Applicant: Affimed GmbH, 68165 Mannheim (DE)
(72) Inventor: Pietzko, Kerstin, 68165 Mannheim (DE); Schütz, Daniel, 68165 Mannheim (DE); Overesch, Andre, 68165 Mannheim (DE); Pahl, Jens, 68165 Mannheim (DE); Dulat, Holger, 68165 Mannheim (DE)
(74) Representative: Schiweck Weinzierl Koch Patentanwälte Partnerschaft mbB

(57) **Abstract**

The present invention relates to a bispecific antibody construct for use in treating non-small cell lung cancer (NSCLC) in a subject, optionally in combination with an immune checkpoint inhibitor, wherein the bispecific antibody construct is administered in a loading dose for a first period of time, and is then administered in a maintenance dose for a second period of time, wherein the loading dose is higher than the maintenance dose. Also provided are compositions comprising an immune checkpoint inhibitor and a bispecific antibody construct and there use in treating non-small cell lung cancer (NSCLC) in a subject and methods of stratifying and treating a tumor patient, comprising determining the type of tumor of the patient, and electing patients diagnosed with NSCLC for treatment with the bispecific antibody construct.

## Description

### Field of the invention

The present invention relates to a bispecific antibody construct for use in a method of treating non-small cell lung cancer (NSCLC) in a subject, wherein the bispecific antibody construct comprises at least one first binding domain capable of specifically binding CD16A and at least one second binding domain capable of specifically binding EGFR, wherein the method comprises administering the bispecific antibody construct in a loading dose for a first period of time, and administering the bispecific antibody construct in a maintenance dose for a second period of time, wherein the loading dose is higher than the maintenance dose. The present invention also relates to a composition comprising an immune checkpoint inhibitor and said bispecific antibody construct, wherein the immune checkpoint inhibitor is present in an amount (by weight) that is in the range of about 1.0 to about 1.3 times, about 1.1 to about 1.2 times, or about 1.15 to about 1.18 times the amount of the bispecific antibody construct. Also provided is the use of said composition in a method of treating non-small cell lung cancer (NSCLC) in a subject according to the method of the invention. Further provided is a bispecific antibody construct comprising at least one first binding domain capable of specifically binding CD16A and at least one second binding domain capable of specifically binding EGFR and optionally an immune checkpoint inhibitor for use in a method of treating NSCLC in a subject and providing an expected median progression free survival of the subject or a group of subjects suffering from NSCLC. The present invention also relates to a method of stratifying and treating a tumor patient comprising determining the type of tumor of the patient, and electing the patient for treatment with a bispecific antibody construct according to the method of the invention. Also provided is an immune checkpoint inhibitor or a combination of an immune checkpoint inhibitor and a bispecific antibody construct, for use in a method of treating non-small cell lung cancer (NSCLC) in a subject in accordance with the method of the invention.

### Background

The treatment of non-small cell lung cancer (NSCLC) in the second and third lines is critical due to several limitations of current therapies. In particular traditional chemotherapy agents like docetaxel and pemetrexed have shown limited efficacy in improving overall survival and quality of life for patients in the second and third lines of treatment (Morabito BMC Medicine (2018) 16:24). The response rates are generally low, and the duration of response is often short. Moreover, many of the currently used chemotherapies are associated with significant toxicity and adverse effects, which can severely impact the patient's quality of life (Favaretto, Europ Oncol (2009) 5(1):38). This is particularly challenging for patients who have already undergone first-line treatments and may have compromised health. Another issue is that tumors often develop resistance to initial treatments, making subsequent therapies less effective (Davies 2017, PLOS one 0175679). This resistance can be due to various genetic and molecular changes in the tumor cells, which necessitates the need for new treatments that can overcome or bypass these resistance mechanisms. While newer therapies such as immunotherapy (e.g., nivolumab, pembrolizumab) and targeted therapies (e.g., EGFR inhibitors) have shown promise, they are not effective for all patients. There is a significant portion of patients who do not respond to these treatments or who experience progression after an initial response. Given these challenges, there is a high medical need for the development of new and more effective treatments for NSCLC patients in the second and third lines of therapy. These treatments should ideally have better efficacy, lower toxicity, and the ability to overcome resistance mechanisms.

Thus, there is an existing need in the art for the provision of improved therapies and respective dosage regimen for treating NSCLC in a subject. The present invention addresses this need as indicated herein.

### Summary

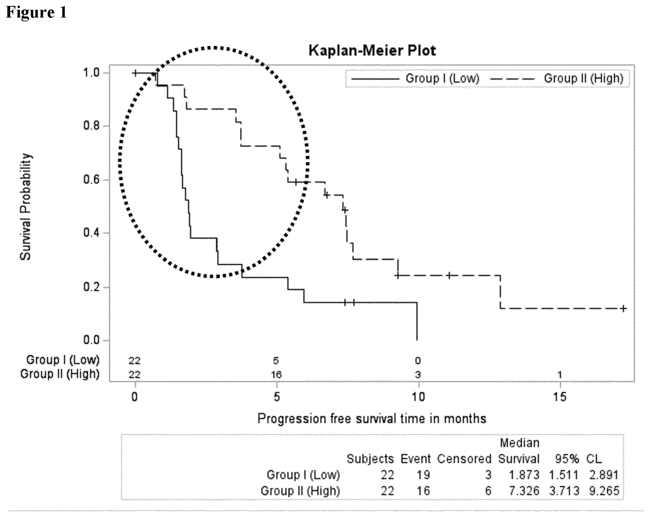

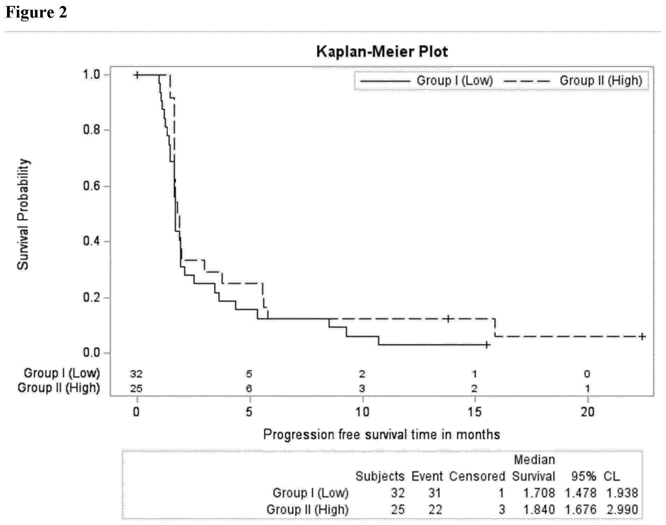

The present invention is based at least partly on the surprising finding that in the treatment of solid tumor patients, it was observed that the plasma concentration of a bispecific antibody construct specifically binding CD16A and EGFR, such as AFM24 is heterogenous among patients. For non-NSCLC solid tumor patients, no significant difference in median progression free survival has been observed between patients having high plasma concentration (high group) or low plasma concentration (low group) of the bispecific antibody construct. Furthermore, there is no difference in the Kaplan Meier Curves overall and especially in the first weeks of treatment **(****Figure 2****)**. However, it was surprisingly found, that for NSCLC patients, the median progression free survival in the patients of the high group is significantly prolonged compared to the patients of the low group and there is drastic difference of the Kaplan Meier Curves overall and especially in the first weeks of treatment **(****Figure 1****)**. Thus, the data received by the inventors of the present invention clearly prove that only NSCLC patients benefit from a high plasma concentration of AFM24 during the first weeks of the treatment while other solid tumor patients do not. Accordingly, it has been surprisingly found that by increasing the dose of AFM24 in the first weeks of treatment, plasma concentrations of AFM24 of the vast majority of patients can be increased to a level that is at least equivalent to the plasma concentrations of AFM24 in the high group of patients during the first weeks of treatment. Thus, the data provided herein strongly indicate that NSCLC patients showing a higher plasma concentration (high Group) of the bispecific antibody construct at the start of therapy have a better prognosis for prolonged progression free survival **(****Figure 1****),** while this is not the case for non-NSCLC solid tumor patients **(****Figure 2****)** or NSCLC patients showing a lower plasma concentration (low Group) of the bispecific antibody construct at the start of therapy **(****Figure 1****).** This finding was surprising and completely unexpected in that one could not assume from the outset that an initial higher dose of a bispecific antibody construct specifically binding CD16A and EGFR is associated with a more favorable course of therapy only for NSCLC patients, but not for other patients suffering from solid tumors. In light of this surprising result, the present invention points to the treatment of all NSCLC patients with an initially higher dose of the bispecific antibody construct in order to increase success of therapy and progression free survival time of said patients.

Accordingly, in one aspect the present invention provides for a bispecific antibody construct for use in a method of treating non-small cell lung cancer (NSCLC) in a subject, wherein the bispecific antibody construct comprises at least one first binding domain capable of specifically binding CD16A and at least one second binding domain capable of specifically binding EGFR, wherein the method comprises administering the bispecific antibody construct in a loading dose for a first period of time, and administering the bispecific antibody construct in a maintenance dose for a second period of time, wherein the loading dose is higher than the maintenance dose.

In some embodiments, the method further comprises administering to the subject an immune checkpoint inhibitor.

In some embodiments, the tumor is characterized by epidermal growth factor receptor (EGFR) overexpression on tumor cells. In some embodiments, the tumor is an EGFR-positive tumor.

In some embodiments, the first binding domain of the bispecific antibody construct binds to an epitope on CD16A which is C-terminal to the physiological Fcy receptor binding domain, said epitope preferably comprising Y158 of SEQ ID NO: 1.

In some embodiments, the first binding domain of the bispecific antibody construct comprises
(i) a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13);
(ii) a VH region comprising CDR-H1 sequence GYTFTSYY (SEQ ID NO: 17), CDR-H2 sequence IEPMYGST (SEQ ID NO: 18), and CDR-H3 sequence ARGSAYYYDFADY (SEQ ID NO: 19), and a VL region comprising CDR-L1 sequence NIGSKN (SEQ ID NO: 20), CDR-L2 sequence QDN, and CDR-L3 sequence QVWDNYSVL (SEQ ID NO: 21);
(iii) a VH region comprising CDR-H1 sequence GYTFTNYY (SEQ ID NO: 25), CDR-H2 sequence INPSGGVT (SEQ ID NO: 26), and CDR-H3 sequence ARGSAYYYDFADY (SEQ ID NO: 27), and a VL region comprising CDR-L1 sequence NIGSKS (SEQ ID NO: 28), CDR-L2 sequence QDK, and CDR-L3 sequence QVWDDYIVL (SEQ ID NO: 29);
(iv) a VH region comprising CDR-H1 sequence SYYMH (SEQ ID NO: 32), CDR-H2 sequence AIEPRYGSTSYAQKFQG (SEQ ID NO: 33), and CDR-H3 sequence GSAYYYDFADY (SEQ ID NO: 34), and a VL region comprising CDR-L1 sequence GGHNIGSKNVH (SEQ ID NO: 35), CDR-L2 sequence QDNKRPS (SEQ ID NO: 36), and CDR-L3 sequence QVWDNYNVL (SEQ ID NO: 37); or
(v) a VH region comprising CDR-H1 sequence NYYMQ (SEQ ID NO: 38), CDR-H2 sequence IINPSGGVTSYAQKFQG (SEQ ID NO: 39), and CDR-H3 sequence GSAYYYDFADY (SEQ ID NO: 40), and a VL region comprising CDR-L1 sequence GGNNIGSKSVH (SEQ ID NO: 41), CDR-L2 sequence QDKKRPS (SEQ ID NO: 42), and a CDR-L3 sequence QVWDDYIVL (SEQ ID NO: 43).

In some embodiments, the first binding domain of the bispecific antibody construct comprises
(i) a VH region having at least 80%, at least 85%, at least 90%, at least 95%, at least 98%, or at least 99% sequence identity to SEQ ID NO: 14 and a VL region having at least 80%, at least 85%, at least 90%, at least 95%, at least 98%, or at least 99% sequence identity to SEQ ID NO: 15;
(ii) a VH region having at least 80%, at least 85%, at least 90%, at least 95%, at least 98%, or at least 99% sequence identity to SEQ ID NO: 22 and a VL region having at least 80%, at least 85%, at least 90%, at least 95%, at least 98%, or at least 99% sequence identity to SEQ ID NO: 23;
(iii) a VH region having at least 80%, at least 85%, at least 90%, at least 95%, at least 98%, or at least 99% sequence identity to SEQ ID NO: 30 and a VL region having at least 80%, at least 85%, at least 90%, at least 95%, at least 98%, or at least 99% sequence identity to SEQ ID NO: 31;
(iv) a VH region having at least 80%, at least 85%, at least 90%, at least 95%, at least 98%, or at least 99% sequence identity to SEQ ID NO: 44 and a VL region having at least 80%, at least 85%, at least 90%, at least 95%, at least 98%, or at least 99% sequence identity to SEQ ID NO: 45;
(v) a VH region having at least 80%, at least 85%, at least 90%, at least 95%, at least 98%, or at least 99% sequence identity to SEQ ID NO: 46 and a VL region having at least 80%, at least 85%, at least 90%, at least 95%, at least 98%, or at least 99% sequence identity to SEQ ID NO: 47;
(vi) a VH region having at least 80%, at least 85%, at least 90%, at least 95%, at least 98%, or at least 99% sequence identity to SEQ ID NO: 48 and a VL region having at least 80%, at least 85%, at least 90%, at least 95%, at least 98%, or at least 99% sequence identity to SEQ ID NO: 49;
(vii) a VH region having at least 80%, at least 85%, at least 90%, at least 95%, at least 98%, or at least 99% sequence identity to SEQ ID NO: 50 and a VL region having at least 80%, at least 85%, at least 90%, at least 95%, at least 98%, or at least 99% sequence identity to SEQ ID NO: 51;
(viii) a VH region having at least 80%, at least 85%, at least 90%, at least 95%, at least 98%, or at least 99% sequence identity to SEQ ID NO: 52 and a VL region having at least 80%, at least 85%, at least 90%, at least 95%, at least 98%, or at least 99% sequence identity to SEQ ID NO: 53; or
(ix) a VH region having at least 80%, at least 85%, at least 90%, at least 95%, at least 98%, or at least 99% sequence identity to SEQ ID NO: 54 and a VL region having at least 80%, at least 85%, at least 90%, at least 95%, at least 98%, or at least 99% sequence identity to SEQ ID NO: 55.

In some embodiments, the first binding domain of the bispecific antibody construct comprises
(i) a VH region as depicted in SEQ ID NO: 14 and a VL region as depicted in SEQ ID NO: 15;
(ii) a VH region as depicted in SEQ ID NO: 22 and a VL region as depicted in SEQ ID NO: 23;
(iii) a VH region as depicted in SEQ ID NO: 30 and a VL region as depicted in SEQ ID NO: 31;
(iv) a VH region as depicted in SEQ ID NO: 44 and a VL region as depicted in SEQ ID NO: 45;
(v) a VH region as depicted in SEQ ID NO: 46 and a VL region as depicted in SEQ ID NO: 47;
(vi) a VH region as depicted in SEQ ID NO: 48 and a VL region as depicted in SEQ ID NO: 49;
(vii) a VH region as depicted in SEQ ID NO: 53 and a VL region as depicted in SEQ ID NO: 50;
(viii) a VH region as depicted in SEQ ID NO: 55 and a VL region as depicted in SEQ ID NO: 51; or
(ix) a VH region as depicted in SEQ ID NO: 52 and a VL region as depicted in SEQ ID NO: 53.

In some embodiments, the first binding domain of the bispecific antibody construct is a variable domain (Fv), a single chain Fv (scFv), a Fab, a single chain diabody (scDb), a diabody (Db) or a double Fab.

In some embodiments, the second binding domain of the bispecific antibody construct comprises a VH and a VL domain of an antibody.

In some embodiments, the second binding domain of the bispecific antibody construct comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

In some embodiments, the second binding domain of the bispecific antibody construct comprises a VH region having at least 80%, at least 85%, at least 90%, at least 95%, at least 98%, or at least 99% sequence identity to SEQ ID NO: 61 and a VL region having at least 80%, at least 85%, at least 90%, at least 95%, at least 98%, or at least 99% sequence identity to SEQ ID NO: 62.

In some embodiments, the second binding domain of the bispecific antibody construct comprises a VH region as depicted in SEQ ID NO: 61 and a VL region as depicted in SEQ ID NO: 62.

In some embodiments, the second binding domain of the bispecific antibody construct is a variable domain (Fv), a single chain Fv (scFv), a Fab, a single chain diabody (scDb), a diabody (Db) or a double Fab.

In some embodiments, the bispecific antibody construct binds to a target cell and an immune effector cell simultaneously.

In some embodiments, the bispecific antibody construct comprises a third domain comprising a half-life extension domain.

In some embodiments, said half-life extension domain comprises a CH2 domain, wherein the Fcy receptor binding domain is silenced.

In some embodiments, said half-life extension domain comprises a CH3 domain.

In some embodiments, the bispecific antibody construct comprises at least one hinge domain and a CH3 domain fused to a CH2 domain in an amino to carboxyl order in the order hinge domain - CH2 domain - CH3 domain.

In some embodiments, the bispecific antibody construct comprises at least two of the hinge domain - CH2 domain - CH3 domain elements.

In some embodiments, the second binding domain of the bispecific antibody construct is fused to the C terminus of a CH3 domain and the first binding domain is fused to the N terminus of a hinge region.

In some embodiments, the bispecific antibody construct is bivalent for the first binding domain and bivalent for the second binding domain.

In some embodiments of the bispecific antibody construct,
(a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13; and
(b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

In some embodiments of the bispecific antibody construct,
(a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13), wherein said first binding domain is a Fab;
(b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60), wherein said second binding domain is a scFv; and
(c) the third binding domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

In some embodiments of the bispecific antibody construct,
(a) the first binding domain comprises a VH region as depicted in SEQ ID NO: 14 and a VL region as depicted in SEQ ID NO: 15; and
(b) the second binding domain comprises a VH region as depicted in SEQ ID NO: 61 and a VL region as depicted in SEQ ID NO: 62.

In some embodiments of the bispecific antibody construct,
(a) the first binding domain comprises a VH region as depicted in SEQ ID NO: 14 and a VL region as depicted in SEQ ID NO: 15, wherein said first binding domain is a Fab;
(b) the second binding domain comprises a VH region as depicted in SEQ ID NO: 61 and a VL region as depicted in SEQ ID NO: 62, wherein said second binding domain is a scFv; and
(c) the third domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

In some embodiments, the bispecific antibody construct comprises or consists of an amino acid sequence having at least 80%, at least 85%, at least 90%, at least 95%, at least 98%, or at least 99% sequence identity to the amino acid sequences set forth in SEQ ID NOs: 63 and 64, SEQ ID NOs: 65 and 66; SEQ ID NO: 67; SEQ ID NOs: 63 and 68; or SEQ ID NOs: 65 and 68.

In some embodiments, the bispecific antibody construct comprises or consists of an amino acid sequence set forth in SEQ ID NOs: 63 and 64, SEQ ID NOs: 65 and 66; SEQ ID NO: 67; SEQ ID NOs: 63 and 68; or SEQ ID NOs: 65 and 68.

In some embodiments, the bispecific antibody construct is AFM24.

In some embodiments, the at least one first binding domain of the bispecific antibody construct comprises means for specifically binding CD16A and the at least one second binding domain of the bispecific antibody construct comprises means for specifically binding EGFR.

In some embodiments, the bispecific antibody construct comprises means for specifically binding CD16A and EGFR.

In some embodiments, the immune checkpoint inhibitor is a PD-L1 or PD-1 inhibitor.

In some embodiments, the immune checkpoint inhibitor is an anti-PD-1 antibody or an anti-PD-L1 antibody.

In some embodiments, the immune checkpoint inhibitor is an anti-PD-L1 antibody.

In some embodiments, the immune checkpoint inhibitor is selected from the group consisting of nivolumab atezolizumab, pembrolizumab cemiplimab, H4H7798N, dostarlimab, durvalumab, pidilizumab, MED10608, BI 754091, PF-0680159, spartalizumab, camrelizumab, JNJ-63723283, MCLA-134, H2M8314N, avelumab, MDX-1105, LY3300054, FAZ053, STI-1014, CX-072, KN035, and CK-301.

In some embodiments, the immune checkpoint inhibitor is selected from the group consisting of nivolumab atezolizumab and pembrolizumab,

In some embodiments, the immune checkpoint inhibitor is atezolizumab.

In some embodiments, the immune checkpoint inhibitor is an antibody comprising means for specifically binding PD-L1.

In some embodiments, the bispecific antibody construct is administered to the subject once every about 6 to about 8 days, or once every about 7 days.

In some embodiments, the loading dose of the bispecific antibody construct is administered at a dose in the range of about 600 to about 1200 mg, about 600 to about 850 mg, about 650 to about 800 mg, about 700 to about 750 mg, or about 720 mg.

In some embodiments, the maintenance dose of the bispecific antibody construct is administered at a dose in the range of about 300 to about 550 mg, about 350 to about 550 mg, about 400 to about 550 mg, about 420 to about 530 mg, about 450 to about 500 mg, or about 480 mg.

In some embodiments, a cycle of administering consists of administering the bispecific antibody construct molecule 4 times, and optionally administering the immune checkpoint inhibitor 2 time, and treatment of the subject is carried out for at least one cycle.

In some embodiments, a cycle of administering is about 4 weeks.

In some embodiments, the loading dose of the bispecific antibody construct is administered for up to about 4 cycles, up to about 3 cycles, about to about 2 cycles, about 1 to about 3 cycles, about 1 to about 2 cycles, or about 2 cycles.

In some embodiments, the loading dose of the bispecific antibody construct is administered for up to about 16 weeks, up to about 12 weeks, about to about 8 weeks, about 4 to about 12 weeks, about 4 to about 10 weeks, about 6 to about 10 weeks, about 9 to about 11 weeks, or about 8 weeks.

In some embodiments, the immune checkpoint inhibitor is administered to the subject once every about 1 to about 3 weeks, once every about 2 weeks, once every about 12 to about 16 days, once every about 13 to about 15 days, or once every about 14 days.

In some embodiments, the immune checkpoint inhibitor is administered at a dose in the range of about 500 to about 1200 mg, about 700 to about 1000 mg, about 800 to about 900 mg, or about 840 mg.

In some embodiments, the immune checkpoint inhibitor is administered in the first and the third week of each cycle.

In some embodiments, the immune checkpoint inhibitor and a dose of the bispecific antibody construct are administered to the subject at the same time, or about the same time.

In some embodiments, a dose the immune checkpoint inhibitor is concurrently administered with a dose of the bispecific antibody construct.

In some embodiments, a dose of the bispecific antibody construct and/or a dose the immune checkpoint inhibitor are administered as split-day dosing on two consecutive days.

In some embodiments, the first dose of the bispecific antibody construct and the dose of immune checkpoint inhibitor during the first cycle are administered as split-day dosing on two consecutive days.

In some embodiments, a lead-in dose of the bispecific antibody construct is administered to the subject about 5 to about 8 days or about 6 to about 7 days prior to a first cycle of administration.

In some embodiments, the lead-in dose is about the same dose as the loading dose.

In some embodiments, the lead-in dose is in the range of about 600 to about 850 mg, about 650 to about 800 mg, about 700 to about 750 mg, or about 720 mg.

In some embodiments, the lead-in dose is administered as a split-dose on two consecutive days.

In some embodiments, the bispecific antibody construct and/or the immune checkpoint inhibitor are administered intravenously.

In some embodiments, administering is carried out for a period of time in the range of about 3 months to about 2 years, or about 3 months to about 18 months, or about 3 months to about 12 months.

In some embodiments, the method comprises assessing tumor regression after each cycle of treatment.

In some embodiments, treating results in about 5% (size) or greater shrinkage of tumor, about 10% or greater shrinkage of tumor, about 20% or greater shrinkage of tumor, about 30% or greater shrinkage of tumor, or about 50% or greater shrinkage of tumor.

In some embodiments, the median trough plasma concentration of the bispecific antibody construct is about 97000 ng/mL.

In some embodiments, the expected median progression free survival of the subject or a group of subjects suffering from NSCLC is at least about 3 months, at least about 3.5 months, at least about 4 months, at least about 4.5 months, at least about 5 months, at least about 5.5 months, at least about 6 months, at least about 6.5 months, or at least about 7 months after begin of the treatment.

In some embodiments, the use comprises prior to treatment stratifying the patient based on the type of the tumor and selecting the patient for treatment as described herein after the patient was diagnosed with NSCLC.

Also provided is a composition comprising an immune checkpoint inhibitor and a bispecific antibody construct that comprises at least one first binding domain capable of specifically binding CD16A and at least one second binding domain capable of specifically binding EGFR, wherein the immune checkpoint inhibitor is present in an amount (by weight) that is in the range of about 1.0 to about 1.3 times, about 1.1 to about 1.2 times, or about 1.15 to about 1.18 times the amount of the bispecific antibody construct.

In some embodiment, the composition is in liquid form and configured for intravenous administration.

In some embodiments, the composition comprises one or more excipients.

Also provided is a composition as described herein for use in a method of treating non-small cell lung cancer (NSCLC) in a subject as described herein.

Further provided is a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of treating NSCLC in a subject and providing an expected median progression free survival of the subject or a group of subjects suffering from NSCLC that is about at least about 3 months, at least about 3.5 months, at least about 4 months, at least about 4.5 months, at least about 5 months, at least about 5.5 months, at least about 6 months, at least about 6.5 months, or at least about 7 months after begin of the treatment. In some embodiments, said use is in a method of treatment as defined herein above.

Also provided is a method of stratifying and treating a tumor patient comprising determining the type of tumor of the patient, and electing the patient for treatment with a bispecific antibody construct as described herein if the patient is diagnosed with NSCLC.

Further provided is an immune checkpoint inhibitor of a combination of an immune checkpoint inhibitor and a bispecific antibody construct, for use in a method of treating non-small cell lung cancer (NSCLC) in a subject, wherein the bispecific antibody construct comprises at least one first binding domain capable of specifically binding CD16A and at least one second binding domain capable of specifically binding EGFR, wherein the method comprises administering the bispecific antibody construct in a loading dose for a first period of time, and administering the bispecific antibody construct in a maintenance dose for a second period of time, wherein the loading dose is higher than the maintenance dose, wherein the method is as defined herein above.

### Brief description of the drawings

**Figure 1****:** Kaplan Meier Plot of Progression-free survival (PFS) for NSCLC Trough Set (NSCLC-TR).
**Figure 2****:** Kaplan Meier Plot of Progression-free survival (PFS) for All Trough Set (All-TR) without NSCLC patients.
**Figure 3****:** Mean (+-SD) AFM24 Plasma Trough Concentrations by treatment dose following intravenous (IV) AFM24 Administration in the AFM24-101 Phase 1.
**Figure 4****:** Cancer Type by Organ Summary for All Trough Set (All-TR).
**Figure 5****:** Safety Summary for NSCLC Trough Set (NSCLC-TR).
**Figure 6****:** Safety Summary for All Trough Set (All-TR) without NSCLC patients.
**Figure 7****:** Safety Summary - 480 mg vs 720 mg vs Overall in the AFM24-101 Phase 1.

### Detailed description

In the treatment of tumor patients, it was observed that the plasma concentration of AFM24 in the initial weeks of treatment is heterogenous among patients. For non-NSCLC patients, no significant difference in median progression free survival has been observed in the treatment benefit between patients having high plasma concentration (high group) or low plasma concentration (low group) of AFM24 in the first eight weeks of treatment could be observed. However, it was surprisingly found, that for NSCLC patients, the median progression free survival in the patients of the high group was significantly prolonged compared to the patients of the low group. The inventors have surprisingly found that only NSCLC patients show benefit from a high plasma concentration of AFM24 during the first weeks of the treatment. It has further been found that by increasing the dose of AFM24 in the first weeks of treatment, plasma concentrations of AFM24 of the vast majority of patients can be increased to a level that is at least equivalent to the plasma concentrations of AFM24 in the high group of patients during the first weeks of treatment. Thus, NSCLC patients who received a higher dose of a bispecific antibody construct at the start of therapy had a better prognosis for tumor treatment, while this was not the case for non-NSCLC patients or NSCLC patients who received a lower dose of a bispecific antibody construct at the start of therapy. This finding was surprising in that one cannot assume from the outset that providing an initial higher dose for a limited time/for limited treatment cycles and subsequently reducing the dose in the maintenance treatment is associated with a more favorable course of therapy only for NSCLC patients. Accordingly, the subsequent reduction of the dose in the maintenance treatment did not negatively affect the said more favorable course of therapy of NSCLC patients that received higher doses of AFM24 in the initial weeks of treatment. In light of this surprising result, all NSCLC patients are treated with an initially higher dose of a bispecific antibody construct in order to "push" them into the conditions of the high group of plasma level concentration to allow all patients quickly benefiting from the therapy.

Thus, the present invention points to the use of high doses of a bispecific antibody construct comprising at least one first binding domain capable of specifically binding CD16A and at least one second binding domain capable of specifically binding EGFR for treating NSCLC patients at the start of therapy which may help to significantly prolong median progression free survival of said patients. This is highly relevant since NSCLC patients generally have a poor prognosis. Therefore, the approach provided by the present invention, i.e. treating NSCLC patients with a high loading dose of the bispecific antibody construct in the first weeks of therapy, followed by the administration of the bispecific antibody construct in a lower maintenance dose for the residual treatment period enables to significantly prolong progression free survival time of difficult to treat NSCLC patients. However, this finding was completely unexpected since it was not predictable that an initial higher dose of a bispecific antibody construct is associated with a more favorable course of therapy specifically in NSCLC patients while patients suffering from other types of solid tumor do not benefit from a high initial dose of the bispecific antibody construct.

Accordingly, as set forth herein above, in one aspect the present invention relates to a bispecific antibody construct for use in a method of treating non-small cell lung cancer (NSCLC) in a subject, wherein the bispecific antibody construct comprises at least one first binding domain capable of specifically binding CD16A and at least one second binding domain capable of specifically binding EGFR, wherein the method comprises administering the bispecific antibody construct in a loading dose for a first period of time, and administering the bispecific antibody construct in a maintenance dose for a second period of time, wherein the loading dose is higher than the maintenance dose.

Also provided is a method of treating non-small cell lung cancer (NSCLC) in a subject, the method comprising administering a bispecific antibody construct comprising at least one first binding domain capable of specifically binding CD16A and at least one second binding domain capable of specifically binding EGFR in a loading dose for a first period of time to said subject, and administering said bispecific antibody construct in a maintenance dose for a second period of time to said subject, wherein the loading dose is higher than the maintenance dose.

Also provided is the use of a bispecific antibody construct comprises at least one first binding domain capable of specifically binding CD16A and at least one second binding domain capable of specifically binding EGFR for the manufacture of a medicament for treating non-small cell lung cancer (NSCLC), wherein the medicament is administered in a loading dose for a first period of time and is administered in a maintenance dose for a second period of time, wherein the loading dose is higher than the maintenance dose

As used herein, the terms "treatment," "treat," and "treating" refer to alleviating, ameliorating, delaying the onset of or inhibiting the progress of non-small cell lung cancer (NSCLC) in a subject. For example, a treatment can result in a reduction in the amount of tumor cells, i.e. the tumor size, or a reduced growth rate. Administration of a therapeutically effective amount of a compound as described elsewhere herein for the treatment of NSCLC will result in a reduction of the amount of tumor cells or a decreased growth rate, a reduction in risk or frequency of reoccurrence, a delay in reoccurrence, a reduction in metastasis, increased progression-free and/or decreased morbidity and mortality, among other things.

As used herein, the terms "alleviating" and "ameliorating" refer to any improvement of the disease state of a patient suffering from NSCLC by the administration of the bispecific antibody construct in accordance with the method of the invention. Such an improvement may also be seen as a slowing or stopping of the progression of the tumor or cancer or metastatic cancer of the patient.

As used herein, the term "inhibition", as it relates to cancer and/or cancer cell proliferation and progress of NSCLC, refers to the inhibition of the growth, division, maturation or viability of cancer cells, and/or causing the death of cancer cells, individually or in aggregate with other cancer cells, by cytotoxicity, nutrient depletion, or the induction of apoptosis.

As used herein, "delaying" the onset or "delaying" reoccurrence of a disease or disorder, or one or more symptoms thereof, means to defer, hinder, slow, retard, stabilize and/or postpone development of the disease, disorder, or symptom thereof. This delay can be of varying lengths of time, depending on the history of the disease and/or subject being treated. As it is evident to one skilled in the art, a sufficient or significant delay can, in effect, encompass prevention, in that the subject does not develop the disease, disorder, or symptom thereof. For example, a method that "delays" development of cancer or reoccurrence of the cancer is a method that reduces the probability of disease development in a given time frame and/or reduces extent of the disease in a given time frame, when compared to not using the described method. Such comparisons may be based on clinical studies, using a statistically significant number of subjects.

In some embodiments, treatment may be administered after one or more symptoms have developed. In other embodiments, treatment may be administered in the absence of symptoms. For example, a treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence.

Also provided herein are methods of enhancing, improving and/or increasing the response to an anticancer therapy, in particular a bispecific antibody construct comprising at least one first binding domain capable of specifically binding CD16A and at least one second binding domain capable of specifically binding EGFR, in a subject suffering from NSCLC, comprising administering the bispecific antibody construct described herein in a loading dose for a first period of time, and administering the bispecific antibody construct in a maintenance dose for a second period of time, wherein the loading dose is higher than the maintenance dose, as described herein.

"Enhancing" or "improving" means in this respect, that responsiveness to therapy, and in particular progression-free survival is augmented when administering the bispecific antibody construct in accordance with the method of the present invention to a subject suffering from NSCLC when compared to treatment with said bispecific antibody construct without administering a loading dose that is higher than the maintenance dose of said bispecific antibody construct.

"Increasing" means in this respect that the administration of the bispecific antibody construct as described herein to patients suffering from NSCLC leads to a higher amount of patients responsive to treatment with said bispecific antibody construct when compared to the amount of patients responsive to treatment with the bispecific antibody construct without administering the respective treatment regimen of the present invention, i.e. without administering a loading dose that is higher than the maintenance dose of said bispecific antibody construct. In the context of the present invention, higher responsiveness to treatment with a bispecific antibody construct refers to an increase of treatment efficacy in said patients, in particular an increased progression-free survival.

As used herein, the term "bispecific antibody construct" refers to an antibody construct which is bispecific, i.e., it comprises at least a first binding domain and a second binding domain, wherein the first binding domain binds to one antigen or target (here: NK cell receptor CD16A), and the second binding domain binds to another antigen or target (here: the target cell surface antigen EGFR).

Examples of bispecific antibody constructs are provided, for example, in WO 2006/125668, WO 2017/064221, WO 2019/175368, WO 2019/198051, WO 2020/043670, WO 2021/130383, and Ellwanger et a. (MAbs. 2019 Jul;11(5):899-918).

A bispecific antibody or an antigen binding fragment thereof may comprise a first binding domain capable of specifically binding CD16A and a second binding domain capable of specifically binding EGFR. The bispecific antibody construct disclosed herein may comprise a CD16A binding domain and a EGFR binding domain.

Given that the antibody constructs as defined in the context of the invention are (at least) bispecific, they do not occur naturally and they are markedly different from naturally occurring products. A "bispecific" antibody construct or immunoglobulin is hence an artificial hybrid antibody or immunoglobulin having at least two distinct binding sides with different specificities. Bispecific antibody constructs can be produced by a variety of methods including fusion of hybridomas or linking of Fab' fragments. See, e.g., Songsivilai & Lachmann, Clin. Exp. Immunol. 79:315- 321 (1990).

As used herein, "CD16A" refers to the activating receptor CD16A, also known as FcyRIIIA, expressed on the cell surface of NK cells. CD16A is an activating receptor triggering the cytotoxic activity ofNK cells. The affinity of antibodies for CD16A directly correlates with their ability to trigger NK cell activation, thus higher affinity towards CD16A reduces the antibody dose required for activation. The antigen-binding site of the antigen-binding protein binds to CD16A, but not to CD16B. For example, an antigen-binding site comprising heavy (VH) and light (VL) chain variable domains binding to CD16A, but not binding to CD16B, may be provided by an antigen binding site which specifically binds to an epitope of CD16A which comprises amino acid residues of the C-terminal sequence SFFPPGYQ (SEQ ID NO: 70) and/or residues Gl30 and/or Y141 of CD16A (SEA ID NO: 71) which are not present in CD16B. In some embodiments, the antigen-binding site for CD16A does not bind to CD16B and binds to the known CD16A allotypes F158 and V158 with similar affinity. Two allelic single nucleotide polymorphisms have been identified in human CD16A altering the amino acid in position 158, which is important for interaction with the hinge region of IgG. The allelic frequencies of the homozygous 158 F/F and the heterozygous 158 V/F alleles are similar within the Caucasian population, ranging between 35 and 52% or 38 and 50%, respectively, whereas the homozygous 158 V/V allele is only found in 10-15% (Lopez-Escamez JA et al .; BMC Med Genet 2011; 12: 2). Activation of NK-cells by this anti-CD16A domain in all patients due to the similar affinity is therefore advantageous. Further CD16A antigen-binding sites comprising heavy and light variable chain domains that bind to CD16A, but not to CD16B are described in WO 2006/125668.

The term "EGFR" refers to the epidermal growth factor receptor (EGFR; ErbB-1; HER1) in humans, including all isoforms or variants described with activation, mutations and implicated in pathophysiological processes. A binding domain specific for EGFR may recognize an epitope in the extracellular domain of the EGFR. A binding domain specific for EGFR may specifically bind to human and cynomolgus EGFR, i.e. the binding domain may be cross-reactive to human and cynomolgus EGFR. The epidermal growth factor receptor (EGFR) is a member of the HER family of receptor tyrosine kinases and consists of four members: EGFR (ErbB1/HER1), HER2/neu (ErbB2), HER3 (ErbB3) and HER4 (ErbB4). Stimulation of the receptor through ligand binding (e.g. EGF, TGFa, HB-EGF, neuregulins, betacellulin, amphiregulin) activates the intrinsic receptor tyrosine kinase in the intracellular domain through tyrosine phosphorylation and promotes receptor homo- or heterodimerization with HER family members. These intracellular phospho-tyrosines serve as docking sites for various adaptor proteins or enzymes including SHC, GRB2, PLCg and PI(3)K/Akt, which simultaneously initiate many signaling cascades that influence cell proliferation, angiogenesis, apoptosis resistance, invasion and metastasis. EGFR can be expressed by a number of different tumor types including lung (particularly non-small cell cancer NSCLC), colorectal (particularly CRC), liver (particularly hepatocellular carcinoma HCC), brain (particularly glioblastoma GBM), kidney/renal (particularly ccRCC), ovarian, breast (particularly TNBC), squamous cell carcinoma (particularly squamous cervical cancer), bladder, head and neck (particularly squamous cell carcinoma of head and neck SCCHN), gastric cancer, esophagus, sarcoma, mesothelioma, and adenocarcinoma.

The term "epitope" refers to a side on an antigen to which a binding domain, such as an antibody or immunoglobulin, or a derivative, fragment or variant of an antibody or an immunoglobulin, specifically binds. An "epitope" is antigenic and thus the term epitope is sometimes also referred to herein as "antigenic structure" or "antigenic determinant". Thus, the binding domain is an "antigen interaction site". Said binding/interaction is also understood to define a "specific recognition". "Epitopes" can be formed both by contiguous amino acids or non-contiguous amino acids juxtaposed by tertiary folding of a protein. A "linear epitope" is an epitope where an amino acid primary sequence comprises the recognized epitope. A linear epitope typically includes at least 3 or at least 4, and more usually, at least 5 or at least 6 or at least 7, for example, about 8 to about 10 amino acids in a unique sequence. A "conformational epitope", in contrast to a linear epitope, is an epitope wherein the primary sequence of the amino acids comprising the epitope is not the sole defining component of the epitope recognized (e.g., an epitope wherein the primary sequence of amino acids is not necessarily recognized by the binding domain). Typically, a conformational epitope comprises an increased number of amino acids relative to a linear epitope.

As used herein, "CD16B" refers to receptor CD16B, also known as FcyRIIIB, expressed on neutrophils and eosinophils. The receptor is glycosylphosphatidyl inositol (GPI) anchored and is understood to not trigger any kind of cytotoxic activity of CD16B positives immune cells.

In some embodiments, the first binding domain of the multispecific antibody construct used in the context of the means and methods of the present invention binds to an epitope on CD16A which is C-terminal to the physiological Fcy receptor binding domain, said epitope preferably comprising Y158 of SEQ ID NO: 1.

The term "binding domain" in connection with the present invention characterizes a domain which is capable of specifically binding to / interacting with / recognizing a given target epitope or a given target site on the target molecules (antigens), e.g. the NK cell receptor antigen CD16A on the surface of the NK cell, and a target cell surface antigen, i.e. EGFR on the tumor cell, respectively. Thus, in some embodiments, the multispecific antibody construct comprises at least a first binding domain capable of specifically binding CD16A on the surface of an immune effector and at least a second binding domain capable of specifically binding EGFR on the surface of a tumor cell.

The structure and/or function of the first binding domain (recognizing CD16A), and preferably also the structure and/or function of the second binding domain (recognizing the target cell surface antigen EGFR), is/are based on the structure and/or function of an antibody, e.g. of a full-length or whole immunoglobulin molecule and/or is/are drawn from the variable heavy chain (VH) and/or variable light chain (VL) domains of an antibody or fragment thereof. Preferably the first binding domain is characterized by the presence of three light chain CDRs (i.e. CDR1, CDR2 and CDR3 of the VL region) and/or three heavy chain CDRs (i.e. CDR1, CDR2 and CDR3 of the VH region). The second binding domain preferably also comprises the minimum structural requirements of an antibody which allow for the target binding. More preferably, the second binding domain comprises at least three light chain CDRs (i.e. CDRl, CDR2 and CDR3 of the VL region) and/or three heavy chain CDRs (i.e. CDR1, CDR2 and CDR3 of the VH region). In some cases, the first and/or second binding domain is produced by or obtainable by phage-display or library screening methods rather than by grafting CDR sequences from a pre-existing (monoclonal) antibody into a scaffold.

The term "variable" refers to the portions of the antibody or immunoglobulin domains that exhibit variability in their sequence and that are involved in determining the specificity and binding affinity of a particular antibody (i.e., the "variable domain(s)"). The pairing of a variable heavy chain (VH) and a variable light chain (VL) together forms a single antigen-binding side.

Variability is not evenly distributed throughout the variable domains of antibodies; it is concentrated in sub-domains of each of the heavy and light chain variable regions. These sub-domains are called "hypervariable regions" or "complementarity determining regions" (CDRs). The more conserved (i.e., non-hypervariable) portions of the variable domains are called the "framework" regions (FRM or FR) and provide a scaffold for the six CDRs in three-dimensional space to form an antigen-binding surface. The variable domains of naturally occurring heavy and light chains each comprise four FRM regions (FR1, FR2, FR3, and FR4), largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding side (see Kabat et al., loc. cit.).

The terms "CDR", and its plural "CDRs", refer to the complementarity determining region of which three make up the binding character of a light chain variable region (CDR-L1, CDR-L2 and CDR-L3) and three make up the binding character of a heavy chain variable region (CDR-H1, CDR-H2 and CDR-H3). CDRs contain most of the residues responsible for specific interactions of the antibody with the antigen and hence contribute to the functional activity of an antibody molecule: they are the main determinants of antigen specificity. The CDR3 of the light chain and, particularly, the CDR3 of the heavy chain may constitute the most important determinants in antigen binding within the light and heavy chain variable regions. In some antibody constructs, the heavy chain CDR3 appears to constitute the major area of contact between the antigen and the antibody. *In vitro* selection schemes in which CDR3 alone is varied can be used to vary the binding properties of an antibody or determine which residues contribute to the binding of an antigen. Hence, CDR3 is typically the greatest source of molecular diversity within the antibody-binding side. H3, for example, can be as short as two amino acid residues or greater than 26 amino acids.

The exact definitional CDR boundaries and lengths are subject to different classification and numbering systems. CDRs may therefore be referred to by IMGT, Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example the IMGT method as described in Lefranc, M.-P., The Immunologist, 7, 132-136 (1999), Kabat (an approach based on cross-species sequence variability), Chothia (an approach based on crystallographic studies of antigen-antibody complexes), and/or MacCallum (Kabat et al., loc. cit; Chothia et al., J. Mol. Biol, 1987, 196: 901 -917; and MacCallum et al., J. Mol. Biol, 1996, 262: 732). Still another standard for characterizing the antigen binding side is the AbM definition used by Oxford Molecular's AbM antibody modeling software. See, e.g., Protein Sequence and Structure Analysis of Antibody Variable Domains. In: Antibody Engineering Lab Manual (Ed.: Duebel, S. and Kontermann, R., Springer-Verlag, Heidelberg). To the extent that two residue identification techniques define regions of overlapping, but not identical regions, they can be combined to define a hybrid CDR. However, the numbering in accordance with the so-called IMGT system or the Kabat system is preferred.

As used herein, the term "target cell surface antigen" refers to an antigenic structure expressed by a cell and which is present at the cell surface such that it is accessible for an antibody construct as described herein. In the context of the present invention, "target cell surface antigen" to which the bispecific antibody constructs described herein binds to is EGFR.

In some embodiments, binding domains are in the form of one or more polypeptides. Such polypeptides may include proteinaceous parts and non-proteinaceous parts (e.g. chemical linkers or chemical cross-linking agents such as glutaraldehyde). Proteins (including fragments thereof, preferably biologically active fragments, and peptides, usually having less than 30 amino acids) comprise two or more amino acids coupled to each other via a covalent peptide bond (resulting in a chain of amino acids).

In some embodiments, binding domains are in the form of one or more polypeptides. Such polypeptides may include proteinaceous parts and non-proteinaceous parts (e.g. chemical linkers or chemical cross-linking agents such as glutaraldehyde). Proteins (including fragments thereof, preferably biologically active fragments, and peptides, usually having less than 30 amino acids) comprise two or more amino acids coupled to each other via a covalent peptide bond (resulting in a chain of amino acids).

Preferably the binding domain which binds to the NK cell receptor antigen (CD16A) and/or the binding domain which binds to the target cell surface antigen EGFR is/are human, humanized or murine derived chimeric binding domains. Antibodies and antibody constructs comprising at least one human binding domain avoid some of the problems associated with antibodies or antibody constructs that possess non-human such as rodent (e.g. murine, rat, hamster or rabbit) variable and/or constant regions. The presence of such rodent derived proteins can lead to the rapid clearance of the antibodies or antibody constructs or can lead to the generation of an immune response against the antibody or antibody construct by a patient. In order to avoid the use of rodent derived antibodies or antibody constructs, human or fully human antibodies/ antibody constructs can be generated through the introduction of human antibody function into a rodent so that the rodent produces fully human antibodies.

In some embodiments, the at least one first binding domain described herein comprises means for specifically binding CD16A and the at least one second binding domain comprises means for specifically binding EGFR. In some embodiments the bispecific antibody construct comprises means for specifically binding CD16A and EGFR.

The term "specifically binding", as used herein means that the binding domain preferentially binds or recognizes the target even when the binding partner is present in a mixture of other molecules or other structures. The binding may be mediated by covalent or non-covalent interactions or a combination of both. In preferred examples, "simultaneous binding to a target cell and an immune effector cell" comprises the physical interaction between the binding domains and their targets on the cells, but preferably also includes the induction of an action mediated by the simultaneous binding of the two cells. Such an action may be an immune effector function of the immune effector cell, such as a cytotoxic effect. "Means" for specifically binding CD16A and EGFR are structures comprised by a binding domain that help to interact with the target molecule. Said structures are preferably one or more polypeptides described elsewhere herein.

The term "antibody construct" refers to a molecule in which the structure and/or function is/are based on the structure and/or function of an antibody, e.g., of a full-length or whole immunoglobulin molecule and/or is/are drawn from the variable heavy chain (VH) and/or variable light chain (VL) domains of an antibody or fragment thereof. An antibody construct is hence capable of binding to its specific target or antigen. Furthermore, the binding region of an antibody construct comprises the minimum structural requirements of an antibody which allow for the target binding. For the first binding domain to CD16A, this minimum requirement is defined by the presence of a VL region comprising the three light chain CDRs (i.e. CDR1, CDR2 and CDR3 of the VL region) and the presence of a VH region comprising the three heavy chain CDRs (i.e. CDR1, CDR2 and CDR3 of the VH region). For the second binding domain to a tumor antigen, this minimum requirement may, e.g., be defined by the presence of at least the three light chain CDRs (i.e. CDR1, CDR2 and CDR3 of the VL region) and/or the three heavy chain CDRs (i.e. CDR1, CDR2 and CDR3 of the VH region), preferably of all six CDRs. An alternative approach to define the minimal structure requirements of an antibody is the definition of the epitope of the antibody within the structure of the specific target, respectively, the protein domain of the target protein composing the epitope region (epitope cluster) or by reference to a specific antibody competing with the epitope of the defined antibody. The antibodies on which the constructs defined in the context of the invention are based include for example monoclonal, recombinant, chimeric, deimmunized, humanized and human antibodies.

The first binding domain of an antibody construct comprises the designated groups of CDRs. Those CDRs can be comprised in the framework of an antibody light chain variable region (VL) and an antibody heavy chain variable region (VH). The second binding domain comprises defined groups of CDRs. Preferably, those CDRs are comprised in the framework of an antibody light chain variable region (VL) and an antibody heavy chain variable region (VH); however, it does not have to comprise both. Fd fragments, for example, have two VH regions and often retain some antigen-binding function of the intact antigen-binding region.

Examples for the format of antibody fragments, antibody variants or binding domains include (1) a Fab fragment, a monovalent fragment having the VL, VH, CL and CH1 domains; (2) a F(ab')₂fragment, a bivalent fragment having two Fab fragments linked by a disulfide bridge at the hinge domain; (3) an Fd fragment having the two VH and CH1 domains; (4) an Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv), the latter being preferred (for example, derived from an scFv-library). Examples for antibody constructs are e.g. described in WO00/006605, WO2005/040220, WO2008/119567, WO2010/037838, WO2013/026837, WO2013/026833, US2014/0308285, US2014/0302037, WO2014/144722, WO2014/151910, and WO2015/048272.

An antibody construct that can be used in methods of the disclosure can comprise a fragment of a full-length antibody, such as VH, VHH, VL, (s)dAb, Fv, Fd, Fab, Fab', F(ab')₂ or "r IgG" ("half antibody"). Antibody constructs can also comprise modified fragments of antibodies, also called antibody variants, such as scFv, di-scFv or bi(s)-scFv, scFv-Fc, scFv-zipper, scFab, Fab₂, Fabs, diabodies, single chain diabodies, tandem diabodies (Tandab's), tandem di-scFv, tandem tri-scFv, "multibodies" such as triabodies or tetrabodies, and single domain antibodies such as nanobodies or single variable domain antibodies comprising merely one variable domain, which might be VHH, VH or VL, that specifically bind an antigen or epitope independently of other V regions or domains.

As used herein, the terms "single-chain Fv," "single-chain antibodies" or "scFv" refer to single polypeptide chain antibody fragments that comprise the variable regions from both the heavy and light chains, but lack the constant regions. Generally, a single-chain antibody further comprises a polypeptide linker between the VH and VL domains which enables it to form the desired structure which would allow for antigen binding. Exemplary linkers for this purpose include glycine serine linkers, which preferably comprises from about 15 to about 30 amino acids. Preferred glycine serine linkers may have one or more repeats of GGS, GGGS (SEQ ID NO: 72), or GGGGS (SEQ ID NO: 5). Such linker preferably comprises 5, 6, 7, 8, 9 and/or 10 repeats of GGS, preferably (GGS)₆ (SEQ ID NO:2, which are preferably used for scFvs having the arrangement VH-VL), or preferably (GGS)₇ (SEQ ID NO: 3, which are preferably used for scFvs having the arrangement VL-VH). Single chain antibodies are discussed in detail by Plueckthun in The Pharmacology of Monoclonal Antibodies, vol. 1 13, Rosenburg and Moore eds. Springer-Verlag, New York, pp. 269-315 (1994). Various methods of generating single chain antibodies are known, including those described in U.S. Pat. Nos. 4,694,778 and 5,260,203; International Patent Application Publication No. WO 88/01649; Bird (1988) Science 242:423-442; Huston et al. (1988) Proc. Natl. Acad. Sci. USA 85:5879-5883; Ward et al. (1989) Nature 334:54454; Skerra et al. (1988) Science 242:1038- 1041. In specific embodiments, single-chain antibodies can also be human, and/or humanized and/or synthetic. The term "bi-scFv" or "ta-scFv" (tandem scFv) as used herein refers to two scFv that are fused together. Such a bi-scFv or ta-scFv may comprise a linker between the two scFv moieties. Generally, the arrangement of the VH and VL domains on the polypeptide chain within each of the scFv may be in any order. This means that the "bi-scFv" of "ta-scFv" can be arranged in the order VH(1)-VL(1)-VH(2)-VL(2), VL(1)-VH(1)-VH(2)-VL(2), VH(1)-VL(1)-VL(2)-VH(2), or VL(1)-VH(1)-VL(2)-VH(2), where (1) and (2) stand for the first and second scFv, respectively. The term "double Fab" as used herein refers to two Fab fragments that are fused together, which are preferably staggered. Here, a first chain of a first Fab is N-terminally fused to a first chain of a second Fab, or a second chain of a first Fab is N-terminally fused to a second chain of a second Fab, or both, the first chain of a first Fab and the second chain of a first Fab are fused to first and second chains of a second Fab, respectively. A linker may be present between the fused chains of the first and second Fab. The first and second chains of the first and second Fab can be individually selected from a light chain-derived chain of a Fab (VL-CL), a heavy chain derived chain of a Fab (VH-CH1), as long as each Fab contains a VH, a VL, a CH1, and a CL. As an illustrative example, the light chain-derived chain of the first Fab can be fused to the light chain derived-chain of the second Fab. As another illustrative example, the heavy chain-derived chain of the first Fab can be fused to the heavy chain derived-chain of the second Fab. As a further illustrative example, the heavy chain-derived chain of the first Fab can be fused to the light chain derived-chain of the second Fab. In some double Fabs, both chains of the two Fabs are fused together. For example, the light chain-derived chain of the first Fab can be fused to the light chain derived-chain of the second Fab while the heavy chain-derived chain of the first Fab can be fused to the heavy chain derived-chain of the second Fab. Alternatively, the light chain-derived chain of the first Fab can be fused to the heavy chain derived-chain of the second Fab while the heavy chain-derived chain of the first Fab can be fused to the light chain derived-chain of the second Fab.

In some embodiments, the binding domain specifically binding CD16A of the bispecific antibody construct comprises a heavy chain variable domain (VH_CD16a) comprising a heavy chain complementarity determining region 1 (CDR-H1) having the sequence GYTFTSYY (SEQ ID NO: 9); a heavy chain complementarity determining region 2 (CDR-H2) having the sequence INPSGGST (SEQ ID NO: 10); and a heavy chain complementarity determining region 3 (CDR-H3) having the sequence ARGSAYYYDFADY (SEQ ID NO: 11), and a light chain variable domain (VL_CD16a) comprising a light chain complementarity determining region 1 (CDR-L1) having the sequence NIGSKN (SEQ ID NO: 12), a light chain complementarity determining region 2 (CDR-L2) having the sequence QDN; and a light chain complementarity determining region 3 (CDR-L3) having the sequence QVWDNYSVL (SEQ ID NO: 13).

In some embodiments, the binding domain specifically binding CD16A of the bispecific antibody construct comprises a heavy chain variable domain (VH_CD16a) comprising a heavy chain complementarity determining region 1 (CDR-H1) having the sequence GYTFTSYY (SEQ ID NO: 17); a heavy chain complementarity determining region 2 (CDR-H2) having the sequence IEPMYGST (SEQ ID NO: 18); and a heavy chain complementarity determining region 3 (CDR-H3) having the sequence ARGSAYYYDFADY (SEQ ID NO: 19), and a light chain variable domain (VL_CD16a) comprising a light chain complementarity determining region 1 (CDR-L1) having the sequence NIGSKN (SEQ ID NO: 20), a light chain complementarity determining region 2 (CDR-L2) having the sequence QDN; and a light chain complementarity determining region 3 (CDR-L3) having the sequence QVWDNYSVL (SEQ ID NO: 21).

In some embodiments, the binding domain specifically binding CD16A of the bispecific antibody construct comprises a heavy chain variable domain (VH_CD16a) comprising a heavy chain complementarity determining region 1 (CDR-H1) having the sequence GYTFTNYY (SEQ ID NO: 25); a heavy chain complementarity determining region 2 (CDR-H2) having the sequence INPSGGVT (SEQ ID NO: 26); and a heavy chain complementarity determining region 3 (CDR-H3) having the sequence ARGSAYYYDFADY (SEQ ID NO: 27), and a light chain variable domain (VL_CD16a) comprising a light chain complementarity determining region 1 (CDR-L1) having the sequence NIGSKS (SEQ ID NO: 28), a light chain complementarity determining region 2 (CDR-L2) having the sequence QDK; and a light chain complementarity determining region 3 (CDR-L3) having the sequence QVWDDYIVL (SEQ ID NO: 29).

In some embodiments, the binding domain specifically binding CD16A of the bispecific antibody construct comprises a heavy chain variable domain (VH_CD16a) comprising a heavy chain complementarity determining region 1 (CDR-H1) having the sequence SYYMH (SEQ ID NO: 32); a heavy chain complementarity determining region 2 (CDR-H2) having the sequence AIEPRYGSTSYAQKFQG (SEQ ID NO: 33); and a heavy chain complementarity determining region 3 (CDR-H3) having the sequence GSAYYYDFADY (SEQ ID NO: 34), and a light chain variable domain (VL_CD16a) comprising a light chain complementarity determining region 1 (CDR-L1) having the sequence GGHNIGSKNVH (SEQ ID NO: 35), a light chain complementarity determining region 2 (CDR-L2) having the sequence QDNKRPS (SEQ ID NO: 36); and a light chain complementarity determining region 3 (CDR-L3) having the sequence QVWDNYNVL (SEQ ID NO: 37).

In some embodiments, the binding domain specifically binding CD16A of the bispecific antibody construct comprises a heavy chain variable domain (VH_CD16a) comprising a heavy chain complementarity determining region 1 (CDR-H1) having the sequence NYYMQ (SEQ ID NO: 38); a heavy chain complementarity determining region 2 (CDR-H2) having the sequence IINPSGGVTSYAQKFQG (SEQ ID NO: 39); and a heavy chain complementarity determining region 3 (CDR-H3) having the sequence GSAYYYDFADY (SEQ ID NO: 40), and a light chain variable domain (VL_CD16a) comprising a light chain complementarity determining region 1 (CDR-L1) having the sequence GGNNIGSKSVH (SEQ ID NO: 41), a light chain complementarity determining region 2 (CDR-L2) having the sequence QDKKRPS (SEQ ID NO: 42); and a light chain complementarity determining region 3 (CDR-L3) having the sequence QVWDDYIVL (SEQ ID NO: 43).

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 14; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 15.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 22; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 23.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 30; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 31.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 44; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 45.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 46; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 47.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 48; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 49.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 50; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 51.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 52; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 53.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 54; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 55.

In some embodiments, the binding domain specifically binding CD16A comprises a heavy chain variable (VH) region comprising or consisting of SEQ ID NO: 14; and a light chain variable (VL) region comprising or consisting of SEQ ID NO: 15.

In some embodiments, the binding domain specifically binding CD16A comprises a heavy chain variable (VH) region comprising or consisting of SEQ ID NO: 22; and a light chain variable (VL) region comprising or consisting of SEQ ID NO: 23.

In some embodiments, the binding domain specifically binding CD16A comprises a heavy chain variable (VH) region comprising or consisting of SEQ ID NO: 30; and a light chain variable (VL) region comprising or consisting of SEQ ID NO: 31.

In some embodiments, the binding domain specifically binding CD16A comprises a heavy chain variable (VH) region comprising or consisting of SEQ ID NO: 44; and a light chain variable (VL) region comprising or consisting of SEQ ID NO: 45.

In some embodiments, the binding domain specifically binding CD16A comprises a heavy chain variable (VH) region comprising or consisting of SEQ ID NO: 46; and a light chain variable (VL) region comprising or consisting of SEQ ID NO: 47.

In some embodiments, the binding domain specifically binding CD16A comprises a heavy chain variable (VH) region comprising or consisting of SEQ ID NO: 48; and a light chain variable (VL) region comprising or consisting of SEQ ID NO: 49.

In some embodiments, the binding domain specifically binding CD16A comprises a heavy chain variable (VH) region comprising or consisting of SEQ ID NO: 50; and a light chain variable (VL) region comprising or consisting of SEQ ID NO: 51.

In some embodiments, the binding domain specifically binding CD16A comprises a heavy chain variable (VH) region comprising or consisting of SEQ ID NO: 52; and a light chain variable (VL) region comprising or consisting of SEQ ID NO: 53.

In some embodiments, the binding domain specifically binding CD16A comprises a heavy chain variable (VH) region comprising or consisting of SEQ ID NO: 54; and a light chain variable (VL) region comprising or consisting of SEQ ID NO: 55.

In some embodiments, the first binding domain is a variable domain (Fv). In some embodiments, the first binding domain is a single chain Fv (scFv). In some embodiments, the first binding domain is a Fab. In some embodiments, the first binding domain is a single chain diabody (scDb). In some embodiments, the first binding domain is a diabody (Db). In some embodiments, the first binding domain is a double Fab. Preferably, the first binding domain is a Fab.

In some embodiments, the binding domain specifically binding EGFR of the bispecific antibody construct, i.e. the second binding domain, comprises a heavy chain variable domain (VH_EGFR) comprising a heavy chain complementarity determining region 1 (CDR-H1) having the sequence GGSVSSGSYY (SEQ ID NO: 56); a heavy chain complementarity determining region 2 (CDR-H2) having the sequence IYYSGST (SEQ ID NO: 57); and a heavy chain complementarity determining region 3 (CDR-H3) having the sequence ARNPISIPAFDI (SEQ ID NO: 58), and a light chain variable domain (VL_ EGFR) comprising a light chain complementarity determining region 1 (CDR-L1) having the sequence NIGSKS (SEQ ID NO: 59), a light chain complementarity determining region 2 (CDR-L2) having the sequence YDS; and a light chain complementarity determining region 3 (CDR-L3) having the sequence QVWDTSSDHVL (SEQ ID NO: 60).

In some embodiments, the binding domain specifically binding EGFR comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 61; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 62.

In some embodiments, the binding domain specifically binding EGFR comprises a heavy chain variable (VH) region comprising or consisting of SEQ ID NO: 61; and a light chain variable (VL) region comprising or consisting of SEQ ID NO: 62.

In some embodiments, the second binding domain is a variable domain (Fv). In some embodiments, the second binding domain is a single chain Fv (scFv). In some embodiments, the second binding domain is a Fab. In some embodiments, the second binding domain is a single chain diabody (scDb). In some embodiments, the second binding domain is a diabody (Db). In some embodiments, the second binding domain is a double Fab. Preferably, the second binding domain is a scFv.

In some embodiments, the bispecific antibody construct binds to a target cell and an immune effector cell simultaneously, as described elsewhere herein.

In some embodiments, the bispecific antibody construct comprises a third domain comprising a half-life extension domain. In some embodiments, the half-life extension domain comprises a CH2 domain, wherein the Fcy receptor binding domain is optionally silenced. In some embodiments, the half-life extension domain comprises a CH3 domain.

As used herein the term "half-life extensions domain" relates to a moiety that prolongs serum half-life of the antibody construct. The half-life extension domain may comprise a portion of an antibody, such as an Fc part of an immunoglobulin, a hinge domain, a CH2 domain, a CH3 domain, and/or a CH4 domain. Although less preferred, a half-life extension domain can also comprise elements that are not comprised in an antibody, such as an albumin binding peptide, an albumin binding protein, or transferrin to name only a few. A half-life extension domain preferably does not have an immune-modulatory function. If a half-life extension domain comprises a hinge, CH2 and/or CH3 domain, the half-life extension domain preferably does not essentially bind to an Fc receptor. This can e.g. be achieved through "silencing" of the Fcy receptor binding domain.

As used herein, "silencing" of the Fc or Fcy receptor binding domain refers to any modification that reduces binding of a CH2 domain to an Fc receptor, in particular an Fcy receptor. Such modification can be done by replacement and/or deletion of one or more amino acids that are involved in Fc(y) receptor-binding. Such mutations are well known in the art and have e.g. been described by Saunders (2019, Front. Immunol. 10:1296). For example, a mutation can be located at any one of the positions 233, 234, 235, 236, 237, 239, 263, 265, 267, 273, 297, 329, and 331. Examples for such mutations are: deletion of Glu 233 -> Pro, Glu 233, Leu 234 -> Phe, Leu 234 -> Ala, Leu 234 -> Gly, Leu 234 -> Glu, Leu 234 -> Val, deletion of Leu 234, Leu 235 -> Glu, Leu 235 -> Ala, Leu 235 -> Arg, Leu 235 -> Phe, deletion of Leu 235, deletion of Gly 236, Gly 237 -> Ala, Ser 239 -> Lys, Val 263 -> Leu, Asp 265 -> Ala, Ser 267 -> Lys, Val 273 -> Glu, Asn 297 -> Gly, Asn 297 -> Ala, Lys 332 -> Ala, Pro 329 -> Gly, Pro 331 -> Ser and combinations thereof. Preferably, such a modification comprises one or both of Leu 234 -> Ala and Leu 235 -> Ala (also known as "LALA" mutation). Preferably, such a modification further comprises a Pro 329 -> Gly mutation, also known as "LALA-PG" mutation (Leu 234 -> Ala, Leu 235 -> Ala, and Pro 329 -> Gly). Preferably, such a modification comprises 1, 2, or 3 of the mutations Leu 234 -> Phe, Leu 235 -> Glu, and Asp 265 -> Ala, more preferably all three of these mutations. The combination Leu 234 -> Phe, Leu 235 -> Glu, and Asp 265 -> Ala, which is a preferred modification in the context of the present invention, is also known as "FEA" mutation. Preferably, such a modification further comprises Asn 297 - > Gly. Such a preferred modification comprises the mutations Leu 234 -> Phe, Leu 235 -> Glu, Asp 265 -> Ala, and Asn 297 -> Gly.

In some embodiments, the half-life extension domain may comprise two CH3 domains. The half-life extension domain may comprise a hinge domain. The half-life extension domain may comprise two hinge domains. The half-life extension domain may comprise a CH2 domain and a CH3 domain. In such a case, the CH2 domain and CH3 domain are preferably fused to each other, preferably in the (amino to carboxyl) order CH2 domain - CH3 domain. The half-life extension domain may comprise a hinge domain and a CH2 domain. In such a case, the hinge domain and the CH2 domain are preferably fused to each other, preferably in the (amino to carboxyl) order hinge domain - CH2 domain. The half-life extension domain may comprise a hinge domain, a CH2 domain, and a CH3 domain. In such a case, the hinge domain, the CH2 domain, and CH3 domain are preferably fused to each other, preferably in the (amino to carboxyl) order hinge domain - CH2 domain - CH3 domain. The half-life extending domain may comprise two hinge domain - CH2 domain elements, two CH2 domain - CH3 domain elements, or two hinge domain - CH2 domain - CH3 domain elements. In such a case the two fusions may be located on two different polypeptide strands. Alternatively, the fusions can be located on the same polypeptide strand. An illustrative example for two hinge domain - CH2 domain - CH3 domain elements that are located on the same polypeptide strand is the "single chain Fc" or "scFc" format. Here, both hinge-CH2-CH3 subunits are fused together via a linker that allows assembly of a Fc domain. A preferred linker for this purpose is a glycine serine linker, which preferably comprises from about 20 to about 40 amino acids. Preferred glycine serine linkers may have one or more repeats of GGS, GGGS (SEQ ID NO: 72), or GGGGS (SEQ ID NO: 5). Such linker preferably comprises 4-8 repeats (e.g. 4, 5, 6, 7, or 8 repeats) of GGGGS. Such a linker is preferably (GGGGS)₆, (SEQ ID NO 8). Further scFc constant domains are known in the art and *inter alia* described in WO 2017/134140.

Generally, the bispecific antibody constructs used in accordance with the present invention can be monovalent, bivalent, trivalent, or have an even higher valency for any one of the first target (CD16A) and the second target (EGFR). The antibody constructs of the disclosure may thus comprise one, two, three, or even more of any one of the first binding domain and the second binding domain. It is preferred for the antibody construct of the invention that it is at least monovalent for the first target (CD16A) and at least monovalent for the second target (EGFR). It is also preferred for the antibody construct of the invention that it is at least monovalent for the first target (CD16A) and bivalent for the second target (EGFR). It is further preferred for the antibody construct of the invention that it is at least bivalent for the first target (CD16A) and at least bivalent for the second target (EGFR). It also preferred for the antibody construct of the invention that it is at least bivalent for the first target (CD16A) and at least trivalent for the second target (EGFR). It also preferred for the antibody construct of the invention that it is at least bivalent for the first target (CD16A) and at least monovalent for the second target (EGFR). Most preferred, the antibody construct of the invention is bivalent for the first target (CD16A) and bivalent for the second target (EGFR).

In some embodiments, the bispecific antibody construct used in accordance with the present invention comprises at least one first binding domain and at least one second binding domain. In some embodiments, the bispecific antibody construct comprises at least one first binding domains and at least two second binding domains. It is further preferred for the bispecific antibody construct that it comprises at least two first binding domains and at least two second binding domains. It is further preferred for the bispecific antibody construct that it comprises at least two first binding domains and at least three second binding domains. It is further preferred for the bispecific antibody construct that it comprises at least two first binding domains and at least one second binding domain. Most preferred, the bispecific antibody construct comprises two first binding domains and two second binding domains.

In some embodiments, the first binding domain or the second binding domain of the bispecific antibody construct may be fused to a constant domain of an antibody via a linker. Such a linker is preferably a short linker, which preferably has a length of about 10 nm or less, preferably about 9 nm or less, preferably about 8 nm or less, preferably about 7 nm or less, preferably about 6 nm or less, preferably about 5 nm or less, preferably about 4 nm or less, or even less. The length of the linker is preferably determined as described by Rossmalen et al Biochemistry 2017, 56, 6565-6574, which also describes suitable linkers that are well known to the skilled person. An example for a suitable linker is a glycine serine linker or a serine linker, which preferably comprise no more than about 75 amino acids, preferably not more than about 50 amino acids. In illustrative example, a suitable linker comprises one or more (e.g. 1, 2, 3, 4, 5, 6, 7, or 8) GGGGS sequences (SEQ ID NO: 5), such as (GGGGS)₄ (SEQ ID NO: 7), (GGGGS)₆ (SEQ ID NO: 8), or preferably (GGGGS)₂ (SEQ ID NO: 6). Other illustrative examples for linkers are shown in SEQ ID NOs: 1-8 and 72.

In some embodiments, the bispecific antibody construct may also comprise additional domains, which are e.g. helpful in the isolation of the molecule or relate to an adapted pharmacokinetic profile of the molecule. Domains helpful for the isolation of an antibody construct may be selected from peptide motives or secondarily introduced moieties, which can be captured in an isolation method, e.g. an isolation column. Non-limiting embodiments of such additional domains comprise peptide motives known as Myc-tag, HAT-tag, HA-tag, TAP-tag, GST-tag, chitin binding domain (CBD-tag), maltose binding protein (MBP-tag), Flag-tag, Strep-tag and variants thereof (e.g. StrepII-tag) and His-tag. All herein disclosed antibody constructs characterized by the identified CDRs may comprise a His-tag domain, which is generally known as a repeat of consecutive His residues in the amino acid sequence of a molecule, preferably of five, and more preferably of six His residues (hexa-histidine). The His-tag may be located e.g. at the N- or C-terminus of the antibody construct, preferably it is located at the C-terminus. Most preferably, a hexa-histidine tag is linked via peptide bond to the C-terminus of the antibody construct according to the invention. Additionally, a conjugate system of PLGA-PEG-PLGA may be combined with a poly-histidine tag for sustained release application and improved pharmacokinetic profile. In some embodiments the histidine tag is a hexa-histidine tag (SEQ ID NO: 73).

Amino acid sequence modifications of the antibody constructs described herein are also contemplated, as long as the minimal structural limitations of the first binding domain of the antibody construct of the present invention are maintained. For example, it may be desirable to improve the binding affinity and/or other biological properties of the antibody construct. Amino acid sequence variants of the antibody constructs are prepared by introducing appropriate nucleotide changes into the antibody constructs nucleic acid, or by peptide synthesis. All of the below described amino acid sequence modifications should result in an antibody construct which still retains the desired biological activity (i.e. at least binding to CD16A and EGFR) of the unmodified parental molecule.

Amino acid modifications include, for example, deletions from, and/or insertions into, and/or substitutions of, residues within the amino acid sequences of the antibody constructs. Any combination of deletion, insertion, and substitution is made to arrive at the final construct, provided that the final construct possesses the desired characteristics. The amino acid changes also may alter post-translational processes of the antibody constructs, such as changing the number or position of glycosylation sites.

Generally, if amino acids are substituted in one or more or all of the CDRs of the heavy and/or light chain, it is preferred that the then-obtained "substituted" sequence is at least 60% or at least 65%, more preferably at least 70% or at least 75%, even more preferably at least 80% or at least 85%, and particularly preferably at least 90% or at least 95% identical to the "original" CDR sequence. This means that it is dependent of the length of the CDR to which degree it is identical to the "substituted" sequence. For example, a CDR having 5 amino acids is preferably at least 80% identical to its substituted sequence in order to have at least one amino acid substituted. Accordingly, the CDRs of the antibody construct may have different degrees of identity to their substituted sequences, e.g., CDRL1 may have at least 80%, while CDRL3 may have at least 90%. Preferred substitutions (or replacements) are conservative substitutions. However, any substitution (including non-conservative substitution) is envisaged as long as the antibody construct retains its capability to bind to CD16A via the first binding domain, and/or to the target cell surface antigen via the second binding domain and/or the CDRs of the second binding domain have an identity to the then substituted sequence (at least 60% or at least 65%, more preferably at least 70% or at least 75%, even more preferably at least 80% or at least 85%, and particularly preferably at least 90% or at least 95% identical to the "original" CDR sequence). Conservative substitutions are shown in **Table 1** under the heading of "preferred substitutions". If such substitutions result in a change in biological activity, then more substantial changes, denominated "exemplary substitutions" in Table 1, or as further described below in reference to amino acid classes, may be introduced and the products screened for a desired characteristic.

**Table 1: Amino acid substitutions**

| **Original** | **Exemplary Substitutions** | **Preferred Substitutions** |
|---|---|---|
| Ala (A) | val, leu, i!e | val |
| Arg (R) | lys, gln, asn | lys |
| Asn (N) | gln, his, asp, lys, arg | gln |
| Asp (D) | glu, asn | glu |
| Cys (C) | ser, ala | ser |
| Gln (Q) | asn, glu | asn |
| Glu (E) | asp, gln | asp |
| Gly (G) | ala | ala |
| His (H) | asn, gln, lys, arg | a rg |
| Ile (I) | leu, val, met, ala, phe | leu |
| Leu (L) | norleucine, ile, va!, met, ala | lie |
| Lys (K) | arg, gln, asn | a rg |
| Met (M) | leu, phe, i!e | leu |
| Phe (F) | leu, val, ile, a!a, tyr | tyr |
| Pro (P) | ala | ala |
| ser (S) | thr | thr |
| Thr (T) | ser | ser |
| Trp (W) | tyr, phe | tyr |
| Tyr (Y) | trp, phe, thr, ser | phe |
| Val (V) | ile, leu, met, phe, ala | leu |

Non-conservative substitutions will entail exchanging a member of one of these classes for another class. Any cysteine residue not involved in maintaining the proper conformation of the antibody construct may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

In preferred embodiments, the second binding domain of the bispecific antibody construct is preferably a scFv fragment that is fused to a C terminus of a Fc domain, preferably via the VH domain of the scFv. Accordingly, the arrangement of the polypeptide chain (from N to C) is preferably ...-CH2-CH3-VH-VL, optionally with a linker between the Fc and the scFv. The first binding domain can be located at any suitable position of the antibody construct. Where the antibody construct comprises a Fc region, the first binding domain can be located N terminal of the Fc region, either directly or linked via at least a part of a hinge domain. Other linkers disclosed herein can also be used to link the first binding domain to the Fc domain. A hinge domain is however preferred for this purpose. The first binding domain can be any suitable structure disclosed herein, while a Fab structure is preferred. The first binding domain and the third domain can be fused to each other in a way, that they form a conventional immunoglobuline. Such immunoglobulin may have two heavy chains and two light chains of an immunoglobulin as described elsewhere herein.

The bispecific antibody construct used in accordance with the present invention is preferably in a format referred to as "scFv-IgAb" herein. Such an antibody construct comprises an immunoglobulin that has one scFv fragment fused to the C terminus of each of the two heavy chains, optionally via a linker, preferably a linker disclosed herein. Said scFvs form the second binding domain (EGFR). Two first binding domains (CD16A) are formed by the binding sites of the immunoglobulin. The scFv-IgAb format may comprise four polypeptide chains, two light chains in the arrangement VL(CD16A)-CL, and two heavy chains each fused to a scFv in the arrangement VH(CD16A)-CH1-hinge-CH2-CH3-VH(EGFR)-VL(EGFR) (or less preferred VH(CD16A)-CH1-hinge-CH2-CH3-VL(EGFR)-VH(EGFR)). Illustrative examples for such antibody constructs are shown in SEQ ID NOs: 63 and 64 or 65 and 66.

The bispecific antibody construct used in accordance with the present invention may comprise an immunoglobulin that has one scFv fragment fused to the C terminus of each of the two heavy chains and to the C terminus of each of the two light chains, optionally via a linker, preferably a linker disclosed herein. Said scFvs form the second binding domain (EGFR). Two first binding domains (CD16A) are formed by the binding sites of the immunoglobulin. The scFv-IgAb format may comprise four polypeptide chains, two light chains in the arrangement VL(CD16A)-CL-VH(EGFR)-VL(EGFR), and two heavy chains each fused to a scFv in the arrangement VH(CD16A)-CH1-hinge-CH2-CH3-VH(EGFR)-VL(EGFR) (or less preferred VH(CD16A)-CH1-hinge-CH2-CH3-VL(EGFR)-VH(EGFR)). Illustrative examples for such antibody constructs are shown in SEQ ID NOs: 63 and 68 or 65 and 68.

The bispecific antibody construct used in accordance with the present invention may be in the form of a single chain diabody. As such, the bispecific antibody construct may comprise one single polypeptide chain, in the arrangement VH(EGFR)-VL(CD16A)-VH(CD16A)-VL(EGFR). An illustrative example for such an antibody construct is shown in SEQ ID NO: 67.

In preferred embodiments, the bispecific antibody construct used in accordance with the present invention is a bispecific antibody construct comprising (a) a first binding domain which is capable of specifically binding CD16A, comprising: a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13); and (b) a second binding domain which is capable of specifically binding EGFR, comprising a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

In preferred embodiments, the bispecific antibody construct used in accordance with the present invention is a bispecific antibody construct comprising (a) a first binding domain comprising a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13), wherein said first binding domain is a Fab; (b) a second binding domain comprising a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60), wherein said second binding domain is a scFv; and (c) a third domain comprising two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

In preferred embodiments, the bispecific antibody construct used in accordance with the present invention is a bispecific antibody construct comprising (a) a first binding domain which is capable of specifically binding CD16A, comprising: a VH region as depicted in SEQ ID NO: 14 and a VL region as depicted in SEQ ID NO: 15; and (b) a second binding domain which is capable of specifically binding EGFR, comprising a VH region as depicted in SEQ ID NO: 61 and a VL region as depicted in SEQ ID NO: 62.

In preferred embodiments, the bispecific antibody construct used in accordance with the present invention is a bispecific antibody construct comprising (a) a first binding domain comprising a VH region as depicted in SEQ ID NO: 14 and a VL region as depicted in SEQ ID NO: 15, wherein said first binding domain is a Fab; (b) a second binding domain comprising a VH region as depicted in SEQ ID NO: 61 and a VL region as depicted in SEQ ID NO: 62, wherein said second binding domain is a scFv; and (c) a third domain comprising two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

In preferred embodiments, the bispecific antibody construct used in accordance with the present invention is an antibody construct comprising or consisting of amino acid sequences having at least 80%, at least 85%, at least 90%, at least 95%, at least 98%, or at least 99% sequence identity to the amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68. Preferably the bispecific antibody construct used in accordance with the present invention is an antibody construct comprising or consisting of amino acid sequences having at least 80%, at least 85%, at least 90%, at least 95%, at least 98%, or at least 99% sequence identity to the amino acid sequences set forth in SEQ ID NOs: 63 and 64.

In preferred embodiments, the bispecific antibody construct used in accordance with the present invention is an antibody construct comprising or consisting of amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68. Preferably the bispecific antibody construct used in accordance with the present invention is an antibody construct comprising or consisting of amino acid sequences set forth in SEQ ID NOs: 63 and 64. In this respect is envisaged that the bispecific antibody construct is preferably AFM24.

In the context of the present invention, the method of administering the bispecific antibody construct as described to a subject in need thereof may further comprises administering an immune checkpoint inhibitor (ICI) to said subject. Immune checkpoint inhibitor are compounds that boost anti-cancer immune responses by targeting immunologic receptors on the surface of T-lymphocytes (Shiravand et al. 2022, Curr Oncol. 29(5):3044-3060). ICIs can be used to treat many types of cancers, including non-small cell lung cancer. ICIs function to block the inactivity of immune cells towards tumor cells. Normally, immune cells are controlled under homeostatic conditions using immune checkpoints which maintain a balance between pro-inflammatory and anti-inflammatory signals. ICIs can promote anti-cancer immune responses, shifting the balance to proinflammatory, by binding to immune inhibitory receptors, such as CTLA-4, PD-1, and PD-L receptors on the surface of T-lymphocytes, and allowing activation of the T-lymphocytes towards the tumor. By blocking the immune inhibitory receptors, the ICIs overcome tumor-mediated immune inhibition, and facilitate a local inflammatory anti-tumor effect.

Generally, an immune checkpoint inhibitor used in accordance with the present invention may be one that binds to CTLA-4, PD-1, or PD-L receptors. In some examples, such an immune checkpoint inhibitor binds to a receptor on the surface of T-lymphocytes, and allowing activation of the T-lymphocytes towards the tumor. Preferably, an immune checkpoint inhibitor that can be used in accordance to the present disclosure is one that targets PD-1/PD-L1, which are immune-checkpoint molecules that inhibit the activation of antigen-presenting cells and T cells (priming phase) and the cytotoxic functions of T cells (effector phase). Preferred immune checkpoint inhibitors are PD-L1 inhibitors and PD-1 inhibitors, with PD-L1 inhibitors being more preferred. The immune checkpoint inhibitor is preferably an antibody or an antibody construct. Hence, preferred immune checkpoint inhibitors include anti-PD-1 antibodies and anti-PD-L1 antibodies. Most preferred are anti-PD-L1 antibodies.

It is envisioned by the present invention, that the immune checkpoint inhibitor may be an antibody that comprises means (e.g., an antigen binding site) for specifically binding PD-1 or PD-L1, preferably PD-L1.

Several anti-PD-1 antibodies and anti-PD-L1 antibodies are known to the skilled person and their use according to the present disclosure is contemplated. Examples of such antibodies are given in the following. Nivolumab (OPDIVO^{™}, Bristol Myers Squibb) is a human IgG4 monoclonal antibody that blocks PD-1. See WO2006121168A1 (Ono Co. LTD.). Atezolizumab (Tecentriq^{™}, Genetech) is a humanized, monoclonal antibody of IgG1 isotype against PD-L1 and is used for the treatment of various cancer types. See WO2010077634A1 (Genetech). Pembrolizumab (Keytruda^{™}, Merck), is a humanized IgG4 isotype anti antibody against the PD-1 receptor. See WO2008156712 A1 (N. V. Organon). Cemiplimab (Libtayo^{™}, Regeneron s, Inc.) is a monoclonal antibody that binds to PD-1. See WO2015112800A1, H4H7798N (Regeneron s, Inc). Dostarlimab (TSR-042, JEMPERLI^{™}, Glaxo Smith Kline) is a humanized, monoclonal antibody of the IgG 4κ isotype that binds to PD-1. Durvalumab (Imfinzi^{™}, Medimmune/AstraZeneca) is a human immunoglobulin G1 kappa (IgG1κ) monoclonal antibody that blocks the interaction of PD-L1 with PD-1.

Other anti-PD-1 antibodies include pidilizumab, MED10608, BI 754091, PF-0680159, spartalizumab, camrelizumab, JNJ-63723283, and MCLA-134.

Other anti-PD-L1 antibodies include H2M8314N, avelumab, MDX-1105, LY3300054, FAZ053, STI-1014, CX-072, KN035, and CK-301.

An immune checkpoint inhibitor that can be used in accordance with the present invention, may be selected from the group consisting of nivolumab, atezolizumab, pembrolizumab cemiplimab, H4H7798N, dostarlimab, durvalumab, pidilizumab, MED 10608, BI 754091, PF-0680159, spartalizumab, camrelizumab, JNJ-63723283, MCLA-134, H2M8314N, avelumab, MDX-1105, LY3300054, FAZ053, STI-1014, CX-072, KN035, and CK-301. Preferably, the immune checkpoint inhibitor is selected from the group consisting of nivolumab atezolizumab, pembrolizumab cemiplimab, H4H7798N, dostarlimab, and durvalumab. More preferably, the immune checkpoint inhibitor is selected from the group consisting of nivolumab atezolizumab and pembrolizumab, Even more preferred, the immune checkpoint inhibitor is atezolizumab.

In the context of the present invention, it is particularly envisaged that the NSCLC tumor is characterized by epidermal growth factor receptor (EGFR) overexpression on tumor cells. Overexpression of EGFR is observed in many cancers and means that the cancer cells express a significantly increased or excessive amount of EGFR on their surface when compared to non-cancerous cells of the same cell type and cell stage, i.e. non-cancerous non-small lung cells of the same cell stage. Accordingly, in some embodiments, the present invention aims at the treatment of tumors comprising non-small lung cancer cells that are characterized by overexpression of EGFR.

Thus, it is envisaged that the tumor treated according to the means and methods provided herein is a EGFR-positive tumor. "EGFR-positive" means in this respect that such a tumor may comprise tumor cells which express or overexpress EGFR. Thus, EGFR can be detected on the surface of EGFR-positive cells obtained from said tumor using measuring techniques known to those skilled in the art.

The terms "detecting", "determining," "measuring," "evaluating," "assessing," "assaying," and "analyzing" are often used interchangeably herein to refer to forms of measurement. The terms include determining if an element is present or not (for example, detection). These terms can include quantitative, qualitative or quantitative and qualitative determinations. Assessing can be relative or absolute. "Detecting the presence of" can include determining the amount of something present in addition to determining whether it is present or absent depending on the context.

Thus, EGFR (over)expressed on non-small lung cancer cells is the tumor cell surface antigen targeted and specifically bound by the means and methods of the present invention, in particular the bispecific antibody construct described herein. The term "tumor cell surface antigen" refers to an antigenic structure expressed by a tumor cell and which is present at the tumor cell surface such that it is accessible for the bispecific antibody construct as described herein. Thus, the tumor cell surface antigen targeted by the bispecific antibody construct in accordance with the present invention is EGFR.

The term "EGFR" refers to the epidermal growth factor receptor (EGFR; ErbB-1; HER1 in humans, including all isoforms or variants described with activation, mutations and implicated in pathophysiological processes. The EGFR antigen-binding site recognizes an epitope in the extracellular domain of the EGFR. In certain embodiments the antigen-binding site specifically binds to human and cynomolgus EGFR. The epidermal growth factor receptor (EGFR) is a member of the HER family of receptor tyrosine kinases and consists of four members: EGFR (ErbB1/HER1), HER2/neu (ErbB2), HER3 (ErbB3) and HER4 (ErbB4). Stimulation of the receptor through ligand binding (e.g. EGF, TGFa, HB-EGF, neuregulins, betacellulin, amphiregulin) activates the intrinsic receptor tyrosine kinase in the intracellular domain through tyrosine phosphorylation and promotes receptor homo- or heterodimerization with HER family members. These intracellular phospho-tyrosines serve as docking sites for various adaptor proteins or enzymes including SHC, GRB2, PLCg and PI(3)K/Akt, which simultaneously initiate many signaling cascades that influence cell proliferation, angiogenesis, apoptosis resistance, invasion and metastasis. As set forth above, EGFR can be expressed by a number of different tumor types including lung, particularly non-small cell lung cancer.

Non-small cell lung cancer (NSCLC), also called non-small cell lung carcinoma, is any type of lung cancer other than small cell lung cancer (SCLC) and is the most common type of lung cancer. Non-small cell lung cancer is a type of cancer that forms in the tissues of the lung. There are several types of non-small cell lung cancer, wherein each type of non-small cell lung cancer has different kinds of cancer cells. The cancer cells of each type grow and spread in different ways. The types of non-small cell lung cancer are named for the kinds of **cells** found in the cancer and how the cells look under a microscope. Squamous cell carcinoma is a type of lung cancer that forms in the thin, flat cells lining the inside of the lungs. This is also called epidermoid carcinoma. Large cell carcinoma is a type of lung cancer that may begin in several types of large cells. Adenocarcinoma is a type of lung cancer that begins in the cells that line the alveoli and make substances such as mucus. Less common types of non-small cell lung cancer include adenosquamous carcinoma, sarcomatoid carcinoma, salivary gland carcinoma, carcinoid tumor, and unclassified carcinoma.

NSCLC generally comprises squamous cell carcinoma, large cell carcinoma, adenosquamous carcinoma, sarcomatoid carcinoma, salivary gland carcinoma, carcinoid tumor, and unclassified carcinoma.

As used herein, the terms "cancer", "malignancy" and "tumor" refer to cells having the capacity for autonomous growth, i.e., an abnormal state or condition characterized by rapidly proliferating cell growth, i.e. pathologic hyperproliferation. The term is meant to include all types of cancerous growths or oncogenic processes, metastatic tissues or malignantly transformed cells, tissues, or organs, irrespective of histopathologic type or stage of invasiveness. In the context of the mean and methods provided herein, the terms "cancer", "malignancy" and "tumor" particularly refer to non-small lung cancer cells. Thus, the means and methods provided herein particularly aim at treating a tumor characterized by pathologic hyperproliferation of non-small lung cancer cells.

A "neoplasm" is an abnormal growth of tissue, usually but not always forming a mass. When also forming a mass, it is commonly referred to as a "tumor". Neoplasms or tumors can be benign, potentially malignant (pre-cancerous), or malignant. Malignant neoplasms are commonly called cancer. They usually invade and destroy the surrounding tissue and may form metastases, i.e., they spread to other parts, tissues or organs of the body. Hence, the term "metastatic cancer" encompasses metastases to other tissues or organs than the one of the original tumor. The means and methods provided by the present invention also refer to the treatment of metastatic NSCLC.

The terms "subject," "individual," or "patient" are often used interchangeably herein. Subjects "suffering from a disease" or those "in need of treatment" include those already with the disorder or disease, as well as those in which onset or reoccurrence of the disorder or disease, or symptoms thereof as described elsewhere herein, is to be prevented or delayed. Suitable patients for the means and methods described herein include those who are suffering from, who have been diagnosed with, or who are suspected of having non-small cell lung cancer. Patients subjected to treatment with a bispecific antibody construct comprising at least one first binding domain capable of specifically binding CD16A and at least one second binding domain capable of specifically binding EGFR, wherein the bispecific antibody construct is first administered in a loading dose for a first period of time, and subsequently administered in a maintenance dose for a second period of time, wherein the loading dose is higher than the maintenance dose, respond well to said treatment. As a result, the progression-free and overall survival of said patients is significantly increased when compared to patients directly (or permanently) treated with the lower (maintenance) dose of the bispecific antibody construct described herein.

The terms "subject in need" or those "in need of treatment" includes those already with the disorder or disease, as well as those in which the disorder or disease is to be prevented. The subject in need or "patient" includes human and other mammalian subjects that receive either prophylactic or therapeutic treatment.

In some embodiments, the subject is a mammal selected from the group consisting of an armadillo, an ass, a bat, a bear, a beaver, a cat, a chimpanzee, a cow, a coyote, a deer, a dog, a dolphin, an elephant, a fox, a panda, a gibbon, a giraffe, a goat, a gopher, a hedgehog, a hippopotamus, a horse, a humpback whale, a jaguar, a kangaroo, a koala, a leopard, a lion, a llama, a lynx, a mole, a monkey, a mouse, a narwhal, an orangutan, an orca, an otter, an ox, a pig, a polar bear, a porcupine, a puma, a rabbit, a raccoon, a rat, a rhinoceros, a sheep, a squirrel, a tiger, a walrus, a weasel, a wolf, a zebra, a goat, a horse, and combinations thereof. In some embodiments, the mammal is a human. Thus, subjects to be treated using the means and methods provided herein include human and other mammalian subjects, who have been diagnosed with or are suspected of having NSCLC. In some embodiments, the subject may be a human who exhibits one or more symptoms associated with NSCLC.

The subject, e.g., the human subject, can be a child, e.g., from or from about 0 to or to about 14 years in age. The subject can be a youth, e.g., from or from about 15 to or to about 24 years in age. The subject can be an adult, e.g., from or from about 25 to or to about 64 years in age. The subject can be a senior, e.g. 65+ years in age.

The term "in vivo" is used to describe an event that takes place in a subject's body. The term "ex vivo" is used to describe an event that takes place outside of a subject's body. An ex vivo assay is not performed on a subject. Rather, it is performed upon a sample separate from a subject. An example of an ex vivo assay performed on a sample is an "in vitro" assay. The term "in vitro" is used to describe an event that takes place contained in a container for holding laboratory reagent such that it is separated from the biological source from which the material is obtained. In vitro assays can encompass cell-based assays in which living or dead cells are employed. In vitro assays can also encompass a cell-free assay in which no intact cells are employed.

NSCLC patients that can be treated using the means and methods described herein include those subjects that have undergone "previous treatment". "Previous treatment" as used herein refers to treatment of NSCLC that occurs prior to the treatment provided by the present invention, i.e. prior to administering a bispecific antibody construct comprising at least one first binding domain capable of specifically binding CD16A and at least one second binding domain capable of specifically binding EGFR, wherein the bispecific antibody construct is first administered in a loading dose for a first period of time, and subsequently administered in a maintenance dose for a second period of time, wherein the loading dose is higher than the maintenance dose, respond well to said treatment, to said patient. For example, the subject may have been diagnosed to suffer from NSCLC. After the previous treatment of NSCLC, the subject may not show satisfactory results, such as a partial unsatisfactory response to treatment.

"Previous treatment" as used herein refers to any tumor treatment that occurs prior to treatment according to the present invention, i.e. prior to administering a bispecific antibody construct specifically binding CD16A and EGFR in a loading dose for a first period of time, and administering the bispecific antibody construct in a maintenance dose for a second period of time, wherein the loading dose is higher than the maintenance dose. For example, the subject may have been diagnosed with NSCLC and may have undergone one or more standard therapies for treating NSCLC when receiving the treatment regimen provided herein. In particular, previous treatment of the NSCLC patient may comprise surgery, chemotherapy, radiation therapy, targeted therapy, or immunotherapy, or any combination thereof. For example, the previous treatment of NSCLC may comprise traditional chemotherapy (e.g. treatment with cisplatin, docetaxel, carboplatin, gemcitabine, or pemetrexed, or combinations thereof).

Previous treatment of NSCLC patient may also comprise administration of at least an immune checkpoint inhibitor (ICI) as described elsewhere herein. After the previous treatment, the subject may not show satisfactory results, such as a partial unsatisfactory response to the ICI treatment, or no observable response to the ICI treatment. The subject may also be one that shows satisfactory results at the onset of treatment, but that becomes less responsive to the treatment after a period of time. The previous ICI treatment can use an ICI that binds to immune inhibitory receptors, such as CTLA-4, PD-1, and PD-L receptors. For example, the subject may have had a previous ICI treatment with an immune checkpoint inhibitor such as ipilimumab, pembrolizumab, nivolumab, cemiplimab, dostarlimab, atezolizumab, durvalumab, and avelumab. See, for example, Lee, J.B., et al. (2022) Immune Checkpoint Inhibitors in 10 Years: Contribution of Basic Research and Clinical Application in Cancer Immunotherapy. Immune Netw. 22(1):e2.

As set forth above, the subject receiving previous treatment may have also been treated with one or more traditional chemotherapeutic drugs. For example, the previous treatment may have used one or more platinum-based drugs such as cisplatin, carboplatin, and oxaliplatin. Patients previously treated with platinum-based drugs may have had their treatment discontinued due to systemic toxicity and drug resistance.

The subject receiving previous treatment may have also been treated with one or members of the taxane class of drugs such as paclitaxel (taxol), docetaxel (taxotere), and cabazitaxel. These types of drugs_disrupt microtubule function, which are critical to cell division, and inhibit the process of cell division by preventing as depolymerization. These drugs are also widely used to treat a variety of solid tumors, such as NSCLC. Patients previously treated with taxane class of drugs may have had their treatment discontinued due to toxicities, such as neutropenia, neuropathy, hypersensitivity, and alopecia.

The subject receiving previous treatment may have also been treated with one or more antimetabolite drugs such as methotrexate, pemetrexed, raltitrexed and pralatrexate. Patients previously treated with taxane class of drugs may have had their treatment discontinued due to side effects such as low blood cell count, fatigue, nausea, and gastrointestinal problems.

In some embodiments, the subject may also be one that shows satisfactory results at the onset of an initial treatment, but becomes less responsive to said treatment after a period of time. Subjects having received previous treatments may have undergone treatments with different drugs, or drug combinations, at different times. For example, some subjects may have undergone two or more therapies using different drugs of drug combinations, wherein the therapies were discontinued as not providing desired results.

Any of the means and methods of treatment provided by the present invention may be used to treat NSCLC at various stages. By way of example, the cancer stage includes but is not limited to early, advanced, locally advanced, remission, refractory, reoccurred after remission and progressive. In some embodiments, the subject is at an early stage of a cancer. In other embodiments, the subject is at an advanced stage of cancer. In various embodiments, the subject has a stage I, stage II, stage III or stage IV cancer. The means and methods described herein can promote reduction or retraction of a tumorous cells, decrease or inhibit cancer cell proliferation, and/or induce, increase or promote tumor cell killing. In some embodiments, the subject is in cancer remission. The means and methods described herein can prevent or delay metastasis or recurrence of cancer.

In certain embodiments, the subject may be a human who is (i) substantially refractory to at least one chemotherapy treatment, or (ii) is in relapse after treatment with chemotherapy, or both (i) and (ii). In some of embodiments, the subject is refractory to at least two, at least three, or at least four chemotherapy treatments (including standard or experimental chemotherapies described elsewhere herein).

In the context of the present invention, responsiveness to therapy can generally be determined by measuring or determining the amount of EGFR-positive non-small lung cancer cells in a sample obtained from the treated NSCLC subject, e.g. a sample obtained by lung biopsy. A high responsiveness of a patient to treatment described herein means that no change or a (significantly) reduced amount of EGFR-positive non-small lung cancer cells can be detected in said sample obtained from the treated subject. A low responsiveness of a patient to treatment described herein means that an increase of the amount of EGFR-positive non-small lung cancer cells can be detected in a sample obtained from the treated subject when compared to the amount of EGFR-positive non-small lung cancer cells obtained from said patient before the respective treatment. The responsiveness of tumors is objectively assessed according to the "Response Evaluation Criteria in Solid Tumors" (RECIST).

Alternative to lung biopsy, responsiveness of NSCLC patients to therapy can also be determined using methods such as chest x-ray (CXR), computed tomography (CT) scan, magnetic resonance imaging (MRI) or positron emission tomography (PET) scan, known to those skilled in the art, thereby comparing the results before the respective treatment with the results obtained after the respective treatment. In this respect responsiveness may be determined by comparing the tumor size before and after treatment. A high responsiveness of a patient to treatment described herein means in this case that no increase in tumor size or a (significantly) reduced tumor size can be detected in the treated patient. A low responsiveness of a patient to treatment described herein means in this case that an increase in tumor size can be detected in the treated patient when compared to the tumor size before the respective treatment. Accordingly, responsiveness to treatment can be determined *in vitro* or *in vivo.*

In the context of the present invention, the bispecific antibody construct comprising at least one first binding domain capable of specifically binding CD16A and at least one second binding domain capable of specifically binding EGFR, may be administered to the patient in form of a pharmaceutical composition, e.g. a pharmaceutical composition described herein. Further, when administering the bispecific antibody construct in combination with an ICI to the patient, the ICI may also be administered in form of a pharmaceutical composition.

The term "pharmaceutical composition" relates to a composition which is suitable for administration to a patient, preferably a human patient. In some embodiments, the pharmaceutical composition administered to said patient comprises one or a plurality of the bispecific antibody construct(s) described herein. In some embodiments, the pharmaceutical composition administered to said patient comprises one or a plurality of the bispecific antibody construct(s) described herein. In some embodiments, the pharmaceutical composition administered to said patient comprises one or a plurality of the ICIs described herein.

Accordingly, in the context of the present invention, the bispecific antibody construct(s) described herein may be comprised in a first pharmaceutical composition and the ICI(s) described herein may be comprised in a second pharmaceutical composition, which may be both administered to said patient in the course of the therapeutic treatment. In some embodiments, the ICI(s) and the bispecific antibody construct(s) are comprised in one pharmaceutical composition that is administered to said subject in the course of the therapeutic treatment.

Preferably, the pharmaceutical composition(s) used in the context of the present invention comprises suitable formulations of one or more (pharmaceutically effective) carriers, stabilizers, excipients, diluents, solubilizers, surfactants, emulsifiers, preservatives and/or adjuvants. Acceptable constituents of the composition are preferably nontoxic to recipients at the dosages and concentrations employed. Pharmaceutical compositions that can be used in the context of the present invention include, but are not limited to, liquid, frozen, and lyophilized compositions. As used herein the language "pharmaceutically acceptable carrier" includes saline, solvents, dispersion media, coatings, antibacterial and antifungal agents, isotonic and absorption delaying agents, and the like, compatible with pharmaceutical administration. The use of such media and agents in pharmaceutical compositions is well known in the art, and the compositions comprising such carriers can be formulated by well-known conventional methods.

Pharmaceutical compositions are typically formulated to be compatible with its intended route of administration. Examples of routes of administration include parenteral, e.g., intravenous, intradermal, subcutaneous, oral, transdermal (topical), transmucosal, and rectal administration. In the context of the means and methods of the present invention it is particularly envisaged that the pharmaceutical composition(s) comprising the ICI(s) and/or the bispecific antibody construct(a) is/are suitable for intravenous administration.

Methods of formulating suitable pharmaceutical compositions are known in the art, see, e.g., Remington: The Science and Practice of Pharmacy, 21st ed., 2005; and the books in the series Drugs and the Pharmaceutical Sciences: a Series of Textbooks and Monographs (Dekker, NY). For example, solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent that delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yield a powder of the active ingredient plus any additional desired ingredient from a previously sterile-filtered solution thereof.

As set forth herein above, the means and methods provided by the present invention may also comprise the combination of the bispecific antibody construct comprising at least one first binding domain capable of specifically binding CD16A and at least one second binding domain capable of specifically binding EGFR and an ICI.

"Combination" means in this respect that the ICI and the bispecific antibody construct comprising at least a CD16A and an EGFR binding domain may be used together in the described therapeutic context, specifically in treating NSCLC. In particular, a combination means that the ICI and the bispecific antibody construct comprising at least a CD16A and an EGFR binding domain may be administered together as part of a therapeutic treatment.

As set forth above, in the context of the present invention, the bispecific antibody described herein, e.g. a pharmaceutical composition comprising the bispecific antibody described herein, may be administered to the patient in combination with an ICI as described herein, e.g., a pharmaceutical composition comprising said ICI as described herein. In some embodiments, the bispecific antibody may be administered together with the ICI as part of one pharmaceutical composition. In some embodiments, the bispecific antibody may be administered separately from the ICI, e.g., as part of a separate pharmaceutical composition.

The bispecific antibody constructs comprising at least a CD16A and an EGFR binding domain as described herein can be administered prior to, subsequent to, or simultaneously with administration of the immune checkpoint inhibitor. In some embodiments, the immune checkpoint inhibitor may be administered prior to, subsequent to, or simultaneously with administration of the bispecific antibody construct.

However, as described herein, the bispecific antibody construct comprising at least a CD16A and an EGFR binding domain may also be used/administered without the immune checkpoint inhibitor for treating NSCLC.

The bispecific antibody construct comprising at least a CD16A and an EGFR binding domain used in the treatment of NSCLC as described herein, as well as the immune checkpoint inhibitor, are delivered to the subject in need usually in a specific dosing scheme.

As set forth herein above, in the context of the present invention the bispecific antibody construct is administered for a first period of time in a loading dose and is then administered for a second period of time in a maintenance dose. The term "loading dose" when used herein refers to the first dose of the bispecific antibody construct that is administered to a subject within the first treatment cycle or the first treatment cycles as described elsewhere herein. The term "maintenance dose" as used herein refers to the second dose of the bispecific antibody construct that is administered to a subject within a subsequent treatment cycle or subsequent treatment cycles as described elsewhere herein. In the context of the present invention, the loading dose in the first treatment cycle(s) is higher than the maintenance dose in the subsequent treatment cycle(s), i.e. the loading dose exceeds the maintenance dose.

In some embodiments, the loading dose of the bispecific antibody construct is administered to the subject within the first and second treatment cycle described herein, and the maintenance dose is administered to the subject within the subsequent treatment cycle(s) described herein, i.e. from the third treatment cycle on for any subsequent treatment cycle. However, also when administering the loading dose of the bispecific antibody construct within the first and second treatment cycle (or even further treatment cycles) and the maintenance dose in any subsequent treatment cycle(s), the loading dose necessarily exceeds the maintenance dose. Thus, it is envisaged that during treatment a higher loading dose of the bispecific antibody constructs is given prior to a lower maintenance dose of the bispecific antibody constructs.

In some embodiments, a cycle of administering the bispecific antibody construct to the subject as described herein is about 4 weeks. Thus, the loading dose and the maintenance dose may be administered several times within one treatment cycle. In some embodiments, a cycle of administering consists of administering the bispecific antibody construct molecule 4 times. i.e. the loading and maintenance dose are both administered 4 times within one treatment cycle.

In some embodiments, the bispecific antibody construct is administered to the subject once every about 6 to about 8 days. In some embodiments, the bispecific antibody construct is administered to the subject once every about 7 days.

In some embodiments, the loading dose of the bispecific antibody construct is administered to the subject at a dose in the range of about 600 to about 1200 mg, such as about 600, 650, 700, 750, 800, 850, 900. 950, 100, 1050, 1100, 1150 or 1200 mg. In some embodiments, the loading dose of the bispecific antibody construct is administered to the subject at a dose in the range of about 600 to about 850 mg, such as about 600, 625, 650, 675, 700, 725, 750, 775, 800, 825 or 850 mg. In some embodiments, the loading dose of the bispecific antibody construct is administered to the subject at a dose in the range of about 700 to about 750 mg, such as about 700, 710, 720, 730, 740 or 750 mg. In some embodiments, the loading dose of the bispecific antibody construct is administered to the subject at a dose of about 720 mg.

In some embodiments, the maintenance dose of the bispecific antibody construct is administered to the subject at a dose in the range of about 300 to about 550 mg, such as about 300, 325, 350, 375, 400, 425, 450, 475, 500, 525, or 550 mg. In some embodiments, the maintenance dose of the bispecific antibody construct is administered to the subject at a dose in the range of about 350 to about 550 mg, such as about 350, 375, 400, 425, 450, 475, 500, 525, or 550 mg. In some embodiments, the maintenance dose of the bispecific antibody construct is administered to the subject at a dose in the range of about 400 to about 550 mg, such as about 400, 410, 420, 430, 440, 450, 460, 470, 480, 500, 510, 520, 530, 540, or 550 mg. In some embodiments, the maintenance dose of the bispecific antibody construct is administered to the subject at a dose in the range of about 420 to about 530 mg, such as about 420, 430, 440, 450, 460, 470, 480, 500, 510, 520, or 530 mg. In some embodiments, the maintenance dose of the bispecific antibody construct is administered to the subject at a dose in the range of about 450 to about 500 mg, such as about 450, 455, 460, 465, 470, 475, 480, 485, 490, 495, or 500 mg. In some embodiments, the maintenance dose of the bispecific antibody construct is administered to the subject at a dose of about 480 mg.

As set forth herein above, the administration of the bispecific antibody construct as described herein may further comprise the administration of an immune checkpoint inhibitor for treating NSCLC.

Thus, also provided is an immune checkpoint inhibitor of a combination of an immune checkpoint inhibitor and a bispecific antibody construct as described herein, for use in a method of treating non-small cell lung cancer (NSCLC) in a subject, wherein the bispecific antibody construct comprises at least one first binding domain capable of specifically binding CD16A and at least one second binding domain capable of specifically binding EGFR, wherein the method comprises administering the bispecific antibody construct in a loading dose for a first period of time, and administering the bispecific antibody construct in a maintenance dose for a second period of time, wherein the loading dose is higher than the maintenance dose, wherein the method of treating NSCLC may comprise any of the means and methods described herein.

In some embodiments, the treatment cycle comprises multiple administrations of the bispecific antibody construct and optionally the immune checkpoint inhibitor as described herein. As part of a treatment cycle, the doses of the bispecific antibody construct and the immune checkpoint inhibitor are, in some cases, administered together (e.g., simultaneously or in succession during a single treatment day). In some cases, the doses of the bispecific antibody construct and the immune checkpoint inhibitor are administered together as part of one pharmaceutical composition. In other cases, the doses of the bispecific antibody construct and the immune checkpoint inhibitor are administered separately (e.g., on different treatment days), i.e. as parts of different pharmaceutical compositions.

In some embodiments, the treatment cycle comprises treatment days spread evenly across the treatment cycle. For example, if the treatment cycle is 6 weeks long (or thereabouts), in some cases the treatment days occur every 1 week (or thereabouts). In some embodiments, the treatment cycle comprises administration of the bispecific antibody construct and optionally the immune checkpoint inhibitor on each treatment day. In some embodiments, the treatment cycle comprises administration of the bispecific antibody construct and optionally the immune checkpoint inhibitor on a subset of the treatment days. In some embodiments, the treatment cycle comprises administration of the bispecific antibody construct and optionally the immune checkpoint inhibitor on each treatment day. In some embodiments, the treatment cycle comprises administration of the bispecific antibody construct and optionally the immune checkpoint inhibitor on a subset of the treatment days. In some embodiments, the bispecific antibody construct and optionally the immune checkpoint inhibitor are administered only during the first half of the treatment cycle.

In some embodiments, a cycle of administering consists of administering the bispecific antibody construct molecule 4 times, and optionally administering the immune checkpoint inhibitor 2 times, and treatment of the subject is carried out for at least one cycle.

In some embodiments, the loading dose of the bispecific antibody is administered for up to about 4 cycles. In some embodiments, the loading dose of the bispecific antibody is administered for up to about 3 cycles. In some embodiments, the loading dose of the bispecific antibody is administered for up to about 2 cycles. In some embodiments, the loading dose of the bispecific antibody is administered for about 1 to about 3 cycles. In some embodiments, the loading dose of the bispecific antibody is administered for about 1 to about 2 cycles. In some embodiments, the loading dose of the bispecific antibody is administered for about 2 cycles. In some embodiments, the loading dose of the bispecific antibody is administered for about 1 cycle.

In some embodiments, the loading dose of the bispecific antibody is administered for up to about 16 weeks. In some embodiments, the loading dose of the bispecific antibody is administered for up to about 12 weeks. In some embodiments, the loading dose of the bispecific antibody is administered for about to about 8 weeks. In some embodiments, the loading dose of the bispecific antibody is administered for about 4 to about 12 weeks. In some embodiments, the loading dose of the bispecific antibody is administered for about 4 to about 10 weeks. In some embodiments, the loading dose of the bispecific antibody is administered for about 6 to about 10 weeks. In some embodiments, the loading dose of the bispecific antibody is administered for about 9 to about 11 weeks. In some embodiments, the loading dose of the bispecific antibody is administered for about 8 weeks.

The maintenance dose may be administered to the subject as long as the patient benefits from treatment. Preferably, the subject is treated with the maintenance dose as long as the patient does not show a progressive disease (PD). In some embodiments, the maintenance dose of the bispecific antibody is administered for up to about 36 cycles or more. In some embodiments, the maintenance dose of the bispecific antibody is administered for up to about 30 cycles or more. In some embodiments, the maintenance dose of the bispecific antibody is administered for up to about 25 cycles or more. In some embodiments, the maintenance dose of the bispecific antibody is administered for up to about 20 cycles or more. In some embodiments, the maintenance dose of the bispecific antibody is administered for up to about 18 cycles or more. In some embodiments, the maintenance dose of the bispecific antibody is administered for up to about 16 cycles or more. In some embodiments, the maintenance dose of the bispecific antibody is administered for up to about 14 cycles or more. In some embodiments, the maintenance dose of the bispecific antibody is administered for up to about 12 cycles or more.

In some embodiments, the maintenance dose of the bispecific antibody is administered for about 10 cycles. In some embodiments, the maintenance dose of the bispecific antibody is administered for about 9 cycles. In some embodiments, the maintenance dose of the bispecific antibody is administered for about 8 cycles. In some embodiments, the maintenance dose of the bispecific antibody is administered for about 7 cycles. In some embodiments, the maintenance dose of the bispecific antibody is administered for about 6 cycles. In some embodiments, the maintenance dose of the bispecific antibody is administered for about 5 cycles. In some embodiments, the maintenance dose of the bispecific antibody is administered for about 4 cycles. In some embodiments, the maintenance dose of the bispecific antibody is administered for about 3 cycles. In some embodiments, the maintenance dose of the bispecific antibody is administered for about 2 cycles. In some embodiments, the maintenance dose of the bispecific antibody is administered for about 1 cycle. In some embodiments, the maintenance dose of the bispecific antibody is administered for about 1 to about 20 cycles. In some embodiments, the maintenance dose of the bispecific antibody is administered for about 1 to about 18 cycles. In some embodiments, the maintenance dose of the bispecific antibody is administered for about 1 to about 16 cycles. In some embodiments, the maintenance dose of the bispecific antibody is administered for about 1 to about 14 cycles. In some embodiments, the maintenance dose of the bispecific antibody is administered for about 1 to about 12 cycles. In some embodiments, the maintenance dose of the bispecific antibody is administered for about 1 to about 10 cycles. In some embodiments, the maintenance dose of the bispecific antibody is administered for about 1 to about 8 cycles. In some embodiments, the maintenance dose of the bispecific antibody is administered for about 1 to about 6 cycles. In some embodiments, the maintenance dose of the bispecific antibody is administered for about 1 to about 4 cycles. In some embodiments, the maintenance dose of the bispecific antibody is administered for about 1 to about 3 cycles. Preferably, the amount of cycles for the maintenance dose exceeds the amount of cycles for the loading dose.

In some embodiments, the bispecific antibody construct as described herein, and optionally the immune checkpoint inhibitor as described herein, are administered to the patient as part of a treatment cycle that spans multiple days. In some embodiments, the bispecific antibody construct and optionally the immune checkpoint inhibitor are administered to the patient as part of a treatment regimen that comprises one or more treatment cycles.

In some embodiments, the dosing schedule can vary over the course of the therapy. Thus, for example, the patient can receive a first series of doses, i.e. the loading dose as described herein, every 1, 2, 3, or 4 weeks and a second series of doses, i.e. the maintenance dose described herein, every 1, 2, 3, or 4 weeks, wherein the time between the first series of doses and the second series of doses is different. In some embodiments, the patient receives 1, 2, 3, 4, 5, 6, 7, 8, 9, 10, 11, 12 or more doses in the first series of doses and 1, 2, 3, 4, 5, 6, 7, 8, 9, 10, 11, 12 or more doses in the second series of doses, wherein the number of doses in the first series and the second series of doses can be the same or different. Thus, for example, a patient may receive eight doses administered every other week in the first series of doses and twelve or more doses administered every week in the second series of doses.

In some embodiments, the treatment regimen comprises a treatment break (e.g., a period without administration of the bispecific antibody construct and/or without administration of the immune checkpoint inhibitor) between treatment cycles. In some embodiments, the treatment break is from 1 to 8 weeks, e.g., from 1 to 8, 1 to 7, 1 to 6, 1 to 5, 1 to 4, 1 to 3, 1 to 2, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, or 7 to 8 weeks. In some embodiments, the treatment break is at least 1, 2, 3, 4, 5, 6, 7, or 8 weeks. In some embodiments, the treatment break is or is about 1, 2, 3, 4, 5, 6, 7, or 8 weeks.

In some embodiments, the treatment regimen continues until the patient's disorder (i.e. the tumor disease) progresses, or until the doses are discontinued due to the patient's intolerance of the bispecific antibody construct. In some embodiments, the treatment regimen continues until the doses are discontinued due to the patient's intolerance of the immune checkpoint inhibitor. In some embodiments, the treatment regimen continues until the doses are discontinued due to the patient's intolerance of all two compounds.

The ability to offer repeat treatment/dosing may allow patients to experience or maintain a deeper or prolonged response from the therapy. Thus, in some embodiments, the patient receives multiple doses, e.g., two, three, four, five, six, seven, eight, nine, ten or more doses of the bispecific antibody construct and optionally of the immune checkpoint inhibitor. In some embodiments, a patient can receive response-based dosing, during which the patient continues to receive the bispecific antibody construct and optionally the immune checkpoint inhibitor for as long as the patient derives a benefit. The number of doses and the number of the bispecific antibody construct, and optionally of the immune checkpoint inhibitor administered in each dose can also be tailored to the individual patient. Thus, the therapies described herein can be tailored to each patient based on that patient's own response. In some cases, the therapy can be terminated if the patient no longer derives a benefit from therapy. In some cases, the therapy can also be reinitiated if the patient relapses.

In some embodiments, the treatment cycle is from 2 to 60 days, e.g., from 2 to 50, 2 to 40, 2 to 30, 2 to 20, 2 to 10, 2 to 5, 5 to 60, 5 to 50, 5 to 40, 5 to 30, 5 to 20, 5 to 10, 10 to 60, 10 to 50, 10 to 40, 10 to 30, 10 to 20, 20 to 60, 20 to 50, 20 to 40, 20 to 30, 30 to 60, 30 to 50, 30 to 40, 40 to 60, 40 to 50, or 50 to 60 days. In some embodiments, the treatment cycle is from 1 to 8 weeks (e.g., 1 to 7, 1 to 6, 1 to 5, 1 to 4, 1 to 3, 1 to 2, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, or 7 to 8 weeks). In some embodiments, the treatment cycle is or is about 1, 2 , 3, 4, 5, 6, 7, or 8 weeks. Preferably, one treatment cycle is about 28 days, i.e. 4 weeks.

In the context of the above-described administration schemes, the length of the treatment cycle(s) selected for administering the loading dose refers to the first period of time of administration of the bispecific antibody construct, and the length of the treatment cycle(s) selected for administering the maintenance dose refers to the second period of time of administration of the bispecific antibody construct.

In the context of the present invention, the treatment cycles of a treatment regimen with respect to the used loading dose and maintenance dose of the bispecific antibody construct are different regarding the dosing of the antibody construct, as described elsewhere herein may. In some embodiments, the treatment cycles of a treatment regimen may be the same with respect to the dosing and timing schedules of the immune checkpoint inhibitor. In some embodiments, the treatment cycles of a treatment regimen may be different with respect to the dosing and timing schedules of the immune checkpoint inhibitor.

In some embodiments, the immune checkpoint inhibitor is administered to the subject once every about 1 to about 3 weeks. In some embodiments, the immune checkpoint inhibitor is administered to the subject once every about 2 weeks. In some embodiments, the immune checkpoint inhibitor is administered to the subject once every about 12 to about 16 days. In some embodiments, the immune checkpoint inhibitor is administered to the subject once every about 13 to about 15 days. In some embodiments, the immune checkpoint inhibitor is administered to the subject once every about 14 days.

In some embodiments, the immune checkpoint inhibitor is administered at a dose in the range of about 500 to about 1200 mg, such as about 500, 550, 600, 650, 700, 750, 800, 850, 900. 950, 100, 1050, 1100, 1150 or 1200 mg. In some embodiments, the immune checkpoint inhibitor is administered at a dose in the range of about 700 to about 1000 mg, such as about 700, 725, 750, 775, 800, 825, 850, 875, 900, 925, 950, 975, or 1000 mg. In some embodiments, the immune checkpoint inhibitor is administered at a dose in the range of about 800 to about 900 mg, such as about 800, 810, 820, 830, 840, 850, 860, 870, 880, 890, or 900 mg. In some embodiments, the immune checkpoint inhibitor is administered at a dose of about 840 mg.

When administering the bispecific antibody construct in combination with an immune checkpoint inhibitor, the used dose of the immune checkpoint inhibitor is preferably envisaged to be higher than the dose of the bispecific antibody construct.

Accordingly, also provided is a (pharmaceutical) composition comprising an immune checkpoint inhibitor and a bispecific antibody construct that comprises at least one first binding domain capable of specifically binding CD16A and at least one second binding domain capable of specifically binding EGFR, wherein the immune checkpoint inhibitor is present in an amount (by weight) that is in the range of about 1.0 to about 1.3 times, about 1.1 to about 1.2 times, or about 1.15 to about 1.18 times the amount of the bispecific antibody construct. In some embodiments, the immune checkpoint inhibitor is present in an amount (by weight) that is in the range of about 1.0 to about 1.3 times, such as about 1.0, 1.025, 1.05, 1.075, 1.1, 1.125, 1.15, 1.175, 1.2, 1.25, 1.225, 1.25, 1.275 or 1.3 times, the amount of the bispecific antibody construct. In some embodiments, the immune checkpoint inhibitor is present in an amount (by weight) that is in the range of about 1.1 to about 1.2 times, such as about 1.1, 1.11, 1.12, 1.13, 1.14, 1.15, 1.16, 1.17, 1.18, 1.19 or 1.2 times, the amount of the bispecific antibody construct. In some embodiments, the immune checkpoint inhibitor is present in an amount (by weight) that is in the range of about 1.15 to about 1.8 times, such as about 1.15, 1.155, 1.16, 1,165, 1.17, 1.175, or 1.18 times, the amount of the bispecific antibody construct. Preferably, said composition is used in a method for treating NSCLC as described elsewhere herein.

In some embodiments, the composition comprises one or more excipients, such as those illustratively described in this section and elsewhere herein. Excipients can be used in the invention in this regard for a wide variety of purposes, such as adjusting physical, chemical, or biological properties of formulations, such as adjustment of viscosity, and or processes of one aspect of the invention to improve effectiveness and or to stabilize such formulations and processes against degradation and spoilage due to, for instance, stresses that occur during manufacturing, shipping, storage, pre-use preparation, administration, and thereafter.

In some embodiments, the composition is in liquid form and configured for intravenous administration. Liquid forms refer to compositions based on certain (sterile) diluents and solvents described elsewhere herein in the context of formulating pharmaceutical compositions that are suitable for injection.

In some embodiments, the immune checkpoint inhibitor is administered in the first and the third week of each cycle. In some embodiments, the bispecific antibody construct is administered in the first, second, third and fourth week of each cycle. Thus, in some embodiments, the bispecific antibody construct is administered at each administration, while the immune checkpoint inhibitor is administered at a subset of the administrations. For example, in some embodiments, the bispecific antibody construct is administered once a week and the immune checkpoint inhibitor is administered every two weeks, i.e. twice per month.

In some embodiments, a dose of the immune checkpoint inhibitor and a dose of the bispecific antibody construct are administered to the subject at the same time, or about the same time.

In some embodiments, a dose of the immune checkpoint inhibitor is concurrently administered with a dose of the bispecific antibody construct.

In some embodiments, a dose of the bispecific antibody molecule and/or a dose of the immune checkpoint inhibitor are administered as split-day dosing on two consecutive days. The term "split-day dosing" refers to the practice of breaking one unit dose of an agent, here the bispecific antibody construct and/or the immune checkpoint inhibitor, into two or more separate doses. For example, when administering the bispecific antibody molecule at a dose of about 720 mg, the dose may be split into two doses of about 360 mg which are administered on two consecutive days. In another example, when administering the immune checkpoint inhibitor at a dose of about 480 mg, the dose may be split into two doses of about 240 mg which are administered on two consecutive days. In some embodiments, only the dose of the bispecific antibody construct is administered as split-day dosing. In some embodiments, only the dose of the immune checkpoint inhibitor is administered as split-day dosing. In some embodiments, the dose of the bispecific antibody construct and the immune checkpoint inhibitor is administered as split-day dosing.

In some embodiments, the first dose of the bispecific antibody molecule, i.e. the loading dose, and the dose of immune checkpoint inhibitor during the first cycle are administered as split-day dosing on two consecutive days.

In some embodiments a lead-in dose of the bispecific antibody construct may be administered to the subject. The term "lead-in dose" when used herein refers to the administration of a first dose of the respective bispecific antibody construct to the subject in need thereof prior to start of the first treatment cycle as defined elsewhere herein, i.e. before administering the loading dose defined elsewhere herein to said subject. In some embodiments, a lead-in dose of the bispecific antibody construct is administered about 5 to about 8 days or about 6 to about 7 days prior to a first cycle of administration (treatment).

In some embodiments, the lead-in dose is about the same as the loading dose. In some embodiments, the lead-in dose is in the range of about 600 to about 850 mg, such as about 600, 625, 650, 675, 700, 725, 750, 775, 800, 825, or 850 mg. In some embodiments, the lead-in dose is in the range of about 420 to about 530 mg, such as about 420, 430, 440, 450, 460, 470, 480, 490, 500, 510, 520, or 530 mg. In some embodiments, the lead-in dose is in the range of about 450 to about 500 mg, such as about 450, 455, 460, 465, 470, 475, 480, 485, 490, 495, or 500 mg. In some embodiments, the lead-in dose is about 480 mg.

In some embodiments, the lead-in dose is administered as split-dose on two consecutive days as described elsewhere herein.

In some embodiments, administering of the bispecific antibody construct and/or the immune checkpoint inhibitor as described herein is carried out for a period of time in the range of about 3 months to about 2 years, such as for a period of about 3, 4, 5, 6, 7, 8, 9, 10, 11, 12, 13, 14, 15, 16, 17, 18, 19, 20, 21, 22, 23, or 24 month. In some embodiments, administering of the bispecific antibody construct and/or the immune checkpoint inhibitor as described herein is carried out for a period of time in the range of about 3 months to about 18 months, such as 3, 4, 5, 6, 7, 8, 9, 10, 11, 12, 13, 14, 15, 16, 17, or 18 months. In some embodiments, administering of the bispecific antibody construct and/or the immune checkpoint inhibitor as described herein is carried out for a period of time in the range of about 3 months to about 12 months, such as 3, 4, 5, 6, 7, 8, 9, 10, 11, or 12 months.

Within the context of the present invention it is also envisaged that the means and methods for treating NSCLC as described herein may comprise prior to treatment a step of stratifying patients based on the type of the tumor and selecting the patient for treatment as defined herein after the patient was diagnosed with NSCLC. That means, solid tumor patients may be stratified prior to treatment and specifically patients diagnosed with NSCLC will be selected for subsequent treatment with the bispecific antibody construct according to the invention, i.e. by administered in a loading dose of the antibody construct for a first period of time, followed by administration of a maintenance dose of the antibody construct for a second period of time, wherein the loading dose is higher than the maintenance dose.

Thus, also provided is a method of stratifying and treating a tumor patient, comprising determining the type of tumor of the patient, and electing the patient for treatment as defined in the present invention if the patient is diagnosed with NSCLC. The term "stratifying" means in this respect that the tumor patients are grouped or divided into disease sub-groups, in particular specific sub-groups of solid tumors with respect to the cancer type by organ, where the specific underlying pathology involved is better defined.

It is further envisaged within the context of the present invention that the means and methods described herein may comprise assessing tumor regression after each cycle of treatment. The described treating can result in measurable shrinkage of tumor, as immune effector cells can migrate into the tumor, bind to the bispecific antibody construct and cause a reduction in tumor mass. As supported by the data provided herein, in NSCLC patients receiving the described therapy a prolonged period of progression free survival can be achieved, revealing that the disease had stabilized. In this respect, tumor regression after each cycle of treatment may be assessed using means and methods generally known in the art to detect or determine responsiveness of a NSCLC patient to therapy. Such methods may e.g. comprise CXR, CT scan, MRI, or PET scan as described elsewhere.

In some embodiments, the expected median progression free survival of the treated subject or a group of subjects suffering from NSCLC is at least about 3 months after begin of the treatment. In some embodiments, the expected median progression free survival of the treated subject or a group of subjects suffering from NSCLC is at least about 3.5 months after begin of the treatment. In some embodiments, the expected median progression free survival of the treated subject or a group of subjects suffering from NSCLC is at least about 4 months after begin of the treatment. In some embodiments, the expected median progression free survival of the treated subject or a group of subjects suffering from NSCLC is at least about 4.5 months after begin of the treatment. In some embodiments, the expected median progression free survival of the treated subject or a group of subjects suffering from NSCLC is at least about 5 months after begin of the treatment. In some embodiments, the expected median progression free survival of the treated subject or a group of subjects suffering from NSCLC is at least about 5.5 months after begin of the treatment. In some embodiments, the expected median progression free survival of the treated subject or a group of subjects suffering from NSCLC is at least about 6 months after begin of the treatment. In some embodiments, the expected median progression free survival of the treated subject or a group of subjects suffering from NSCLC is at least about 6.5 months after begin of the treatment. In some embodiments, the expected median progression free survival of the treated subject or a group of subjects suffering from NSCLC is at least about 7 months after begin of the treatment.

Thus, also provided is a bispecific antibody construct and optionally an immune checkpoint inhibitor as described herein, wherein the bispecific antibody construct comprises at least one first binding domain capable of specifically binding CD16A and at least one second binding domain capable of specifically binding EGFR, for use in a method of treating NSCLC in a subject and providing an expected median progression free survival of the subject or a group of subjects suffering from NSCLC that is about at least about 3 months, at least about 3.5 months, at least about 4 months, at least about 4.5 months, at least about 5 months, at least about 5.5 months, at least about 6 months, at least about 6.5 months, or at least about 7 months after begin of the treatment. In this context, the method of treating NSCLC in a subject and providing an expected median progression free survival of the subject or a group of subjects suffering from NSCLC may comprise any of the means and methods described herein for treating NSCLC.

In some embodiments, treatment of NSCLC patients with the above-described methods and administration schemes results in about 5% (size) or greater shrinkage of tumor. In some embodiments, treatment of NSCLC patients with the above-described methods and administration schemes results in about 10% or greater shrinkage of tumor. In some embodiments, treatment of NSCLC patients with the above-described methods and administration schemes results in about 20% or greater shrinkage of tumor. In some embodiments, treatment of NSCLC patients with the above-described methods and administration schemes results in about 30% (size) or greater shrinkage of tumor. In some embodiments, treatment of NSCLC patients with the above-described methods and administration schemes results in about 50% (size) or greater shrinkage of tumor.

In some embodiments, the achieved tumor shrinkage is achieved after two treatment cycles. In some embodiments, the achieved tumor shrinkage is achieved after three treatment cycles. In some embodiments, the achieved tumor shrinkage is achieved after four or more treatment cycles. In some embodiments, the achieved tumor shrinkage is achieved after 4 to 8 treatment cycles. In some embodiments, the achieved tumor shrinkage is achieved after about 4 to 15, about 5 to 12, or about 6 to 9 treatment cycles.

In some embodiments, the median trough plasma concentration of the bispecific antibody construct is about 97000 ng/mL.

Generally, the above-described methods and administration schemes include administering a therapeutically effective amount of the bispecific antibody construct and the immune checkpoint inhibitor. A therapeutic effective amount or dosage of the used the bispecific antibody construct and immune checkpoint inhibitor preferably results in a decrease in severity of disease symptoms, an increase in frequency or duration of disease symptom-free periods or a prevention of impairment or disability due to the disease affliction. The term "effective dose" or "effective dosage" is defined as an amount sufficient to achieve or at least partially achieve beneficial or desired results. For example, a therapeutic amount is one that achieves the desired therapeutic effect. The term "therapeutically effective dose" is defined as an amount sufficient to cure or at least partially arrest the disease and its complications in a patient already suffering from the disease. An effective amount can be administered in one or more administrations, applications or dosages. The compositions can be administered one from one or more times per day to one or more times per week, including once every other day. Amounts or doses effective for this use will depend on the therapeutic context and objectives, the severity of the disease, prior therapy, the patient's clinical history and response to the therapeutic agent, other drugs being administered concurrently, the route of administration, the size (body weight, body surface area, age and sex,) and/or condition (general health) of the patient, and the general state of the patient's own immune system. The proper dose can be adjusted according to the judgment of the attending physician such that it can be administered to the patient once or over a series of administrations, and in order to obtain the optimal therapeutic effect.

Dosage, toxicity and therapeutic efficacy of the therapeutic compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

Therapeutically effective doses of the bispecific antibody construct and the immune checkpoint inhibitor applicable in the context of the presented invention are described elsewhere herein. In some embodiments, a "therapeutically effective dose" of the bispecific antibody construct and the immune checkpoint inhibitor can be defined as the dose increasing the number of NSCLC patients high responsive to treatment as described elsewhere herein.

The bispecific antibody construct and/or the immune checkpoint inhibitor can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, the bispecific antibody construct is administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the bispecific antibody construct. In some embodiments, a given dose is administered by multiple bolus administrations of the bispecific antibody construct, for example, over a period of no more than 3 days, or by continuous infusion administration of the bispecific antibody construct.

In preferred embodiments, the bispecific antibody construct and/or the immune checkpoint inhibitor, or the composition comprising the bispecific antibody construct and/or the immune checkpoint inhibitor, are administered intravenously.

If the pharmaceutical composition comprising the bispecific antibody construct has been lyophilized, the lyophilized material is first reconstituted in an appropriate liquid prior to administration. The lyophilized material may be reconstituted in, e.g., bacteriostatic water for injection (BWFI), physiological saline, phosphate buffered saline (PBS), or the same formulation the protein had been in prior to lyophilization.

Pharmaceutical compositions described herein may be administered using a medical device. In some embodiments, the pharmaceutical compositions are administered intravenously via syringe. Examples of medical devices for administering pharmaceutical compositions are described in U.S. Patent Nos. 4,475,196; 4,439,196; 4,447,224; 4,447, 233; 4,486,194; 4,487,603; 4,596,556; 4,790,824; 4,941,880; 5,064,413; 5,312,335; 5,312,335; 5,383,851; and 5,399,163.

In some embodiments, administration of the bispecific antibody construct and optionally the immune checkpoint inhibitor as described herein, as well as any further combination therapy, is carried out via outpatient delivery.
The invention is further characterized by the following items.

Item 1. A bispecific antibody construct for use in a method of treating non-small cell lung cancer (NSCLC) in a subject, wherein the bispecific antibody construct comprises at least one first binding domain capable of specifically binding CD 16A and at least one second binding domain capable of specifically binding EGFR, wherein the method comprises administering the bispecific antibody construct in a loading dose for a first period of time, and administering the bispecific antibody construct in a maintenance dose for a second period of time, wherein the loading dose is higher than the maintenance dose.

Item 2. The bispecific antibody construct for the use of item 1, wherein the method further comprises administering to the subject an immune checkpoint inhibitor.

Item 3. The bispecific antibody construct for the use of item 1 or 2, wherein the tumor is characterized by epidermal growth factor receptor (EGFR) overexpression on tumor cells.

Item 4. The bispecific antibody construct for the use of any one of the preceding items, wherein the tumor is an EGFR-positive tumor.

Item 5. The bispecific antibody construct for the use of any one of the preceding items, wherein the first binding domain binds to an epitope on CD16A which is C-terminal to the physiological Fcy receptor binding domain, said epitope preferably comprising Y158 of SEQ ID NO: 1.

Item 6. The bispecific antibody construct for the use of any one of the preceding items, wherein the first binding domain comprises
(i) a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13);
(ii) a VH region comprising CDR-H1 sequence GYTFTSYY (SEQ ID NO: 17), CDR-H2 sequence IEPMYGST (SEQ ID NO: 18), and CDR-H3 sequence ARGSAYYYDFADY (SEQ ID NO: 19), and a VL region comprising CDR-L1 sequence NIGSKN (SEQ ID NO: 20), CDR-L2 sequence QDN, and CDR-L3 sequence QVWDNYSVL (SEQ ID NO: 21);
(iii) a VH region comprising CDR-H1 sequence GYTFTNYY (SEQ ID NO: 25), CDR-H2 sequence INPSGGVT (SEQ ID NO: 26), and CDR-H3 sequence ARGSAYYYDFADY (SEQ ID NO: 27), and a VL region comprising CDR-L1 sequence NIGSKS (SEQ ID NO: 28), CDR-L2 sequence QDK, and CDR-L3 sequence QVWDDYIVL (SEQ ID NO: 29);
(iv) a VH region comprising CDR-H1 sequence SYYMH (SEQ ID NO: 32), CDR-H2 sequence AIEPRYGSTSYAQKFQG (SEQ ID NO: 33), and CDR-H3 sequence GSAYYYDFADY (SEQ ID NO: 34), and a VL region comprising CDR-L1 sequence GGHNIGSKNVH (SEQ ID NO: 35), CDR-L2 sequence QDNKRPS (SEQ ID NO: 36), and CDR-L3 sequence QVWDNYNVL (SEQ ID NO: 37); or
(v) a VH region comprising CDR-H1 sequence NYYMQ (SEQ ID NO: 38), CDR-H2 sequence IINPSGGVTSYAQKFQG (SEQ ID NO: 39), and CDR-H3 sequence GSAYYYDFADY (SEQ ID NO: 40), and a VL region comprising CDR-L1 sequence GGNNIGSKSVH (SEQ ID NO: 41), CDR-L2 sequence QDKKRPS (SEQ ID NO: 42), and a CDR-L3 sequence QVWDDYIVL (SEQ ID NO: 43).

Item 7. The bispecific antibody construct for the use of any one of the preceding items, wherein the first binding domain comprises a
(i) a VH region having at least 80%, at least 85%, at least 90%, at least 95%, at least 98%, or at least 99% sequence identity to SEQ ID NO: 14 and a VL region having at least 80%, at least 85%, at least 90%, at least 95%, at least 98%, or at least 99% sequence identity to SEQ ID NO: 15;
(ii) a VH region having at least 80%, at least 85%, at least 90%, at least 95%, at least 98%, or at least 99% sequence identity to SEQ ID NO: 22 and a VL region having at least 80%, at least 85%, at least 90%, at least 95%, at least 98%, or at least 99% sequence identity to SEQ ID NO: 23;
(iii) a VH region having at least 80%, at least 85%, at least 90%, at least 95%, at least 98%, or at least 99% sequence identity to SEQ ID NO: 30 and a VL region having at least 80%, at least 85%, at least 90%, at least 95%, at least 98%, or at least 99% sequence identity to SEQ ID NO: 31;
(iv) a VH region having at least 80%, at least 85%, at least 90%, at least 95%, at least 98%, or at least 99% sequence identity to SEQ ID NO: 44 and a VL region having at least 80%, at least 85%, at least 90%, at least 95%, at least 98%, or at least 99% sequence identity to SEQ ID NO: 45;
(v) a VH region having at least 80%, at least 85%, at least 90%, at least 95%, at least 98%, or at least 99% sequence identity to SEQ ID NO: 46 and a VL region having at least 80%, at least 85%, at least 90%, at least 95%, at least 98%, or at least 99% sequence identity to SEQ ID NO: 47;
(vi) a VH region having at least 80%, at least 85%, at least 90%, at least 95%, at least 98%, or at least 99% sequence identity to SEQ ID NO: 48 and a VL region having at least 80%, at least 85%, at least 90%, at least 95%, at least 98%, or at least 99% sequence identity to SEQ ID NO: 49;
(vii) a VH region having at least 80%, at least 85%, at least 90%, at least 95%, at least 98%, or at least 99% sequence identity to SEQ ID NO: 50 and a VL region having at least 80%, at least 85%, at least 90%, at least 95%, at least 98%, or at least 99% sequence identity to SEQ ID NO: 51;
(viii) a VH region having at least 80%, at least 85%, at least 90%, at least 95%, at least 98%, or at least 99% sequence identity to SEQ ID NO: 52 and a VL region having at least 80%, at least 85%, at least 90%, at least 95%, at least 98%, or at least 99% sequence identity to SEQ ID NO: 53; or
(ix) a VH region having at least 80%, at least 85%, at least 90%, at least 95%, at least 98%, or at least 99% sequence identity to SEQ ID NO: 54 and a VL region having at least 80%, at least 85%, at least 90%, at least 95%, at least 98%, or at least 99% sequence identity to SEQ ID NO: 55.

Item 8. The bispecific antibody construct for the use of any one of the preceding items, wherein the first binding domain comprises a
(i) a VH region as depicted in SEQ ID NO: 14 and a VL region as depicted in SEQ ID NO: 15;
(ii) a VH region as depicted in SEQ ID NO: 22 and a VL region as depicted in SEQ ID NO: 23;
(iii) a VH region as depicted in SEQ ID NO: 30 and a VL region as depicted in SEQ ID NO: 31;
(iv) a VH region as depicted in SEQ ID NO: 44 and a VL region as depicted in SEQ ID NO: 45;
(v) a VH region as depicted in SEQ ID NO: 46 and a VL region as depicted in SEQ ID NO: 47;
(vi) a VH region as depicted in SEQ ID NO: 48 and a VL region as depicted in SEQ ID NO: 49;
(vii) a VH region as depicted in SEQ ID NO: 53 and a VL region as depicted in SEQ ID NO: 50;
(viii) a VH region as depicted in SEQ ID NO: 55 and a VL region as depicted in SEQ ID NO: 51; or
(ix) a VH region as depicted in SEQ ID NO: 52 and a VL region as depicted in SEQ ID NO: 53.

Item 9. The bispecific antibody construct for the use of any one of the preceding items, wherein the first binding domain is a variable domain (Fv), a single chain Fv (scFv), a Fab, a single chain diabody (scDb), a diabody (Db) or a double Fab.

Item 10. The bispecific antibody construct for the use of any one of the preceding items, wherein the second binding domain comprises a VH and a VL domain of an antibody.

Item 11. The bispecific antibody construct for the use of any one of the preceding items, wherein the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

Item 12. The bispecific antibody construct for the use of any one of the preceding items, wherein the second binding domain comprises a VH region having at least 80%, at least 85%, at least 90%, at least 95%, at least 98%, or at least 99% sequence identity to SEQ ID NO: 61 and a VL region having at least 80%, at least 85%, at least 90%, at least 95%, at least 98%, or at least 99% sequence identity to SEQ ID NO: 62.

Item 13. The bispecific antibody construct for the use of any one of the preceding items, wherein the second binding domain comprises a VH region as depicted in SEQ ID NO: 61 and a VL region as depicted in SEQ ID NO: 62.

Item 14. The bispecific antibody construct for the use of any one of the preceding items, wherein the second binding domain is a variable domain (Fv), a single chain Fv (scFv), a Fab, a single chain diabody (scDb), a diabody (Db) or a double Fab.

Item 15. The bispecific antibody construct for the use of any one of the preceding items, wherein the bispecific antibody construct binds to a target cell and an immune effector cell simultaneously.

Item 16. The bispecific antibody construct for the use of any one of the preceding items, wherein the bispecific antibody construct further comprises a third domain comprising a half-life extension domain.

Item 17. The bispecific antibody construct for the use of any one of the preceding items, wherein said half-life extension domain comprises a CH2 domain, wherein the Fcγ receptor binding domain is silenced.

Item 18. The bispecific antibody construct for the use of any one of the preceding items, wherein said half-life extension domain comprises a CH3 domain.

Item 19. The bispecific antibody construct for the use of any one of the preceding items, wherein the ICE comprise at least one hinge domain and a CH3 domain fused to a CH2 domain in an amino to carboxyl order in the order hinge domain - CH2 domain - CH3 domain.

Item 20. The bispecific antibody construct for the use of any one of the preceding items, wherein the antibody construct comprises at least two of the hinge domain - CH2 domain - CH3 domain elements.

Item 21. The bispecific antibody construct for the use of any one of the preceding items, wherein the second binding domain is fused to the C terminus of a CH3 domain and the first binding domain is fused to the N terminus of a hinge region.

Item 22. The bispecific antibody construct for the use of any one of the preceding items, wherein the antibody construct is bivalent for the first binding domain and bivalent for the second binding domain.

Item 23. The bispecific antibody construct for the use of any one of the preceding items, wherein
(a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13; and
(b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

Item 24. The bispecific antibody construct for the use of any one of the preceding items, wherein
(a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13), wherein said first binding domain is a Fab;
(b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60), wherein said second binding domain is a scFv; and
(c) the third domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

Item 25. The bispecific antibody construct for the use of any one of the preceding items, wherein
(a) the first binding domain comprises a VH region as depicted in SEQ ID NO: 14 and a VL region as depicted in SEQ ID NO: 15; and
(b) the second binding domain comprises a VH region as depicted in SEQ ID NO: 61 and a VL region as depicted in SEQ ID NO: 62.

Item 26. The bispecific antibody construct for the use of any one of the preceding items, wherein
(a) the first binding domain comprises a VH region as depicted in SEQ ID NO: 14 and a VL region as depicted in SEQ ID NO: 15, wherein said first binding domain is a Fab;
(b) the second binding domain comprises a VH region as depicted in SEQ ID NO: 61 and a VL region as depicted in SEQ ID NO: 62, wherein said second binding domain is a scFv; and
(c) the third domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

Item 27. The bispecific antibody construct for the use of any one of the preceding items, wherein the antibody construct comprises or consists of amino acid sequences having at least 80%, at least 85%, at least 90%, at least 95%, at least 98%, or at least 99% sequence identity to the amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68.

Item 28. The bispecific antibody construct for the use of any one of the preceding items, wherein the antibody construct comprises or consists of amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68.

Item 29. The bispecific antibody construct for the use of any one of the preceding items, wherein the bispecific antibody construct is AFM24.

Item 30. The bispecific antibody construct for the use of any one of the preceding items, wherein the at least one first binding domain of the bispecific antibody construct comprises means for specifically binding CD16A and the at least one second binding domain of the bispecific antibody construct comprises means for specifically binding EGFR.

Item 31. The bispecific antibody construct for the use of any one of the preceding items, wherein the bispecific antibody construct comprises means for specifically binding CD16A and EGFR.

Item 32. The bispecific antibody construct for the use of any one of the preceding items, wherein the immune checkpoint inhibitor is a PD-L1 or PD-1 inhibitor.

Item 33. The bispecific antibody construct for the use of any one of the preceding items, wherein the immune checkpoint inhibitor is an anti-PD-1 antibody or an anti-PD-L1 antibody.

Item 34. The bispecific antibody construct for the use of any one of the preceding items, wherein the immune checkpoint inhibitor is an anti-PD-L1 antibody.

Item 35. The bispecific antibody construct for the use of any one of the preceding items, wherein the immune checkpoint inhibitor is selected from the group consisting of nivolumab, atezolizumab, pembrolizumab cemiplimab, H4H7798N, dostarlimab, durvalumab, pidilizumab, MED10608, BI 754091, PF-0680159, spartalizumab, camrelizumab, JNJ-63723283, MCLA-134, H2M8314N, avelumab, MDX-1105, LY3300054, FAZ053, STI-1014, CX-072, KN035, and CK-301.

Item 36. The bispecific antibody construct for the use of any one of the preceding items, wherein the immune checkpoint inhibitor is selected from the group consisting of nivolumab atezolizumab and pembrolizumab, preferably wherein the immune checkpoint inhibitor is atezolizumab.

Item 37. The bispecific antibody construct for the use of any one of the preceding items, wherein the immune checkpoint inhibitor is an antibody comprising means for specifically binding PD-L1.

Item 38. The bispecific antibody construct for the use of any one of the preceding items, wherein the bispecific antibody construct is administered to the subject once every about 6 to about 8 days, or once every about 7 days.

Item 39. The bispecific antibody construct for the use of any one of the preceding items, wherein the loading dose of the bispecific antibody construct is administered at a dose in the range of about 600 to about 1200 mg, about 600 to about 850 mg, about 650 to about 800 mg, about 700 to about 750 mg, or about 720 mg.

Item 40. The bispecific antibody construct for the use of any one of the preceding items, wherein the maintenance dose of the bispecific antibody construct is administered at a dose in the range of about 300 to about 550 mg, about 350 to about 550 mg, about 400 to about 550 mg, about 420 to about 530 mg, about 450 to about 500 mg, or about 480 mg.

Item 41. The bispecific antibody construct for the use of any one of the preceding items, wherein a cycle of administering consists of administering the bispecific antibody construct molecule 4 times, and optionally administering the immune checkpoint inhibitor 2 time, and treatment of the subject is carried out for at least one cycle.

Item 42. The bispecific antibody construct for the use of any one of the preceding items, wherein a cycle of administering is about 4 weeks.

Item 43. The bispecific antibody construct for the use of any one of the preceding items, wherein the loading dose of the bispecific antibody is administered for up to about 4 cycles, up to about 3 cycles, about to about 2 cycles, about 1 to about 3 cycles, about 1 to about 2 cycles, or about 2 cycles.

Item 44. The bispecific antibody construct for the use of any one of the preceding items, wherein the loading dose of the bispecific antibody is administered for up to about 16 weeks, up to about 12 weeks, about to about 8 weeks, about 4 to about 12 weeks, about 4 to about 10 weeks, about 6 to about 10 weeks, about 9 to about 11 weeks, or about 8 weeks.

Item 45. The bispecific antibody construct for the use of any one of the preceding items, wherein the immune checkpoint inhibitor is administered to the subject once every about 1 to about 3 weeks, once every about 2 weeks, once every about 12 to about 16 days, once every about 13 to about 15 days, or once every about 14 days.

Item 46. The bispecific antibody construct for the use of any one of the preceding items, wherein the immune checkpoint inhibitor is administered at a dose in the range of about 500 to about 1200 mg, about 700 to about 1000 mg, about 800 to about 900 mg, or about 840 mg.

Item 47. The bispecific antibody construct for the use of any one of the preceding items, wherein the immune checkpoint inhibitor is administered in the first and the third week of each cycle.

Item 48. The bispecific antibody construct for the use of any one of the preceding items, wherein a dose the immune checkpoint inhibitor and a dose of the bispecific antibody construct are administered to the subject at the same time, or about the same time.

Item 49. The bispecific antibody construct for the use of any one of the preceding items, wherein a dose the immune checkpoint inhibitor is concurrently administered with a dose of the bispecific antibody construct.

Item 50. The bispecific antibody construct for the use of any one of the preceding items, wherein a dose of the bispecific antibody molecule and/or a dose the immune checkpoint inhibitor are administered as split-day dosing on two consecutive days.

Item 51. The bispecific antibody construct for the use of any one of the preceding items, wherein the first dose of the bispecific antibody molecule and the dose of immune checkpoint inhibitor during the first cycle are administered as split-day dosing on two consecutive days.

Item 52. The bispecific antibody construct for the use of any one of the preceding items, wherein a lead-in dose of the bispecific antibody construct is administered to the subject about 5 to about 8 days or about 6 to about 7 days prior to a first cycle of administration.

Item 53. The bispecific antibody construct for the use of any one of the preceding items, wherein the lead-in dose is about the same dose as the loading dose.

Item 54. The bispecific antibody construct for the use of any one of the preceding items, wherein the lead-in dose is in the range of about 600 to about 850 mg, about 650 to about 800 mg, about 700 to about 750 mg, or about 720 mg.

Item 55. The bispecific antibody construct for the use of any one of the preceding items, wherein the safety lead-in dose is administered as a split-dose on two consecutive days.

Item 56. The bispecific antibody construct for the use of any one of the preceding items, wherein the bispecific antibody construct and/or the immune checkpoint inhibitor are administered intravenously.

Item 57. The bispecific antibody construct for the use of any one of the preceding items, wherein administering is carried out for a period of time in the range of about 3 months to about 2 years, or about 3 months to about 18 months, or about 3 months to about 12 months.

Item 58. The bispecific antibody construct for the use of any one of the preceding items, wherein the method comprises assessing tumor regression after each cycle of treatment.

Item 59. The bispecific antibody construct for the use of any one of the preceding items, wherein treating results in about 5% (size) or greater shrinkage of tumor, about 10% or greater shrinkage of tumor, about 20% or greater shrinkage of tumor, about 30% or greater shrinkage of tumor, or about 50% or greater shrinkage of tumor.

Item 60. The bispecific antibody construct for the use of any one of the preceding items, wherein the median trough plasma concentration of the bispecific antibody construct is about 97000 ng/mL.

Item 61. The bispecific antibody construct for the use of any one of the preceding items, wherein the expected median progression free survival of the subject or a group of subjects suffering from NSCLC is at least about 3 months, at least about 3.5 months, at least about 4 months, at least about 4.5 months, at least about 5 months, at least about 5.5 months, at least about 6 months, at least about 6.5 months, or at least about 7 months after begin of the treatment.

Item 62. The bispecific antibody construct for the use of any one of the preceding items, wherein the use comprises prior to treatment stratifying the patient based on the type of the tumor and selecting the patient for treatment as defined in any one of items 1-61 after the patient was diagnosed with NSCLC.

Item 63. A composition comprising an immune checkpoint inhibitor and a bispecific antibody construct that comprises at least one first binding domain capable of specifically binding CD16A and at least one second binding domain capable of specifically binding EGFR, wherein the immune checkpoint inhibitor is present in an amount (by weight) that is in the range of about 1.0 to about 1.3 times, about 1.1 to about 1.2 times, or about 1.15 to about 1.18 times the amount of the bispecific antibody construct.

Item 64. The composition of item 63, wherein the composition is in liquid form and configured for intravenous (IV) administration.

Item 65. The composition of item 63 or 64, comprising one or more excipients.

Item 66. The composition of any one of items 63-65 for use in a method as defined in any one of items 1-62.

Item 67. A bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of treating NSCLC in a subject and providing an expected median progression free survival of the subject or a group of subjects suffering from NSCLC that is about at least about 3 months, at least about 3.5 months, at least about 4 months, at least about 4.5 months, at least about 5 months, at least about 5.5 months, at least about 6 months, at least about 6.5 months, or at least about 7 months after begin of the treatment.

Item 68. The bispecific antibody construct for use of item 67, wherein the use is in a method as defined in any one of items 1-62.

Item 69. A method of stratifying and treating a tumor patient comprising determining the type of tumor of the patient, and electing the patient for treatment as defined in any one of items 1-62 if the patient is diagnosed with NSCLC.

Item 70. An immune checkpoint inhibitor or a combination of an immune checkpoint inhibitor and a bispecific antibody construct, for use in a method of treating non-small cell lung cancer (NSCLC) in a subject, wherein the bispecific antibody construct comprises at least one first binding domain capable of specifically binding CD16A and at least one second binding domain capable of specifically binding EGFR, wherein the method comprises administering the bispecific antibody construct in a loading dose for a first period of time, and administering the bispecific antibody construct in a maintenance dose for a second period of time, wherein the loading dose is higher than the maintenance dose, wherein the method is as defined in any one of items 2-62.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 10%, preferably within 5%, more preferably within 2%, even more preferably within 1% of a given value or range (plus (+) or minus (-)). It includes, however, also the concrete number, e.g., about 20 includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein, any of the terms "comprising", "consisting essentially of" and "consisting of" may be replaced with either of the other two terms. For example, the disclosure of the term "comprising" includes the disclosure of the terms "consisting essentially of' as well as the disclosure of the term "consisting of".

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

A better understanding of the present invention and of its advantages will be obtained from the following examples, offered for illustrative purposes only. The examples are not intended to limit the scope of the present invention in any way.

### Examples

### Material & Methods:

A preliminary post-hoc exposure response (ER) analyses was performed that included patients across AFM24-101 and AFM24-102, AFM24-103 treated with AFM24 480 mg as a monotherapy or any combination with atezolizumab/NK Cells **(All-TR,** N=101), and a subset of patients with NSCLC treated with AFM24 as a monotherapy or in combination with atezolizumab **(NSCLC-TR,** N=44).

A mean trough value per patient *(Tpat)* was calculated using all trough values available from C1D22 onwards. Based on the median of these mean trough values in the total population (Median), the patients were split into LOW Trough (Tpat ≤ *Median)* and HIGH Trough (*Tpat > Median*). The objective of this ER analyses was to investigate the association of response (safety, efficacy) endpoints with exposure (PK) measurements. The endpoints assessed were Overall Response Rate (ORR), Disease Control Rate (DCR), Progression-free survival (PFS), and Safety. Only local disease assessments assessed by RECIST v. 1.1 were used. All time to event analyses (PFS) were conducted using Kaplan-Meier Methods.

All data was analysed according to the groups defined above. All statistical analyses are exploratory in a statistical sense even if they may use confirmatory methods.

### Results:

All patients included in the analyses outlined above were treated with 480 mg AFM24.

### All-TR:

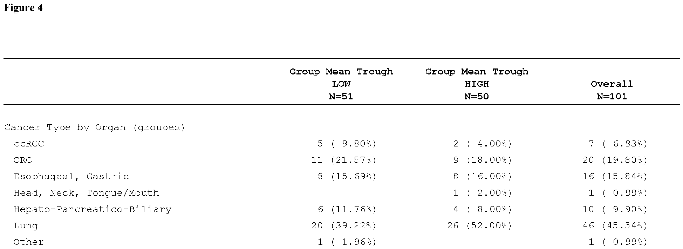

The median threshold for the All-TR Group was: 86460 ng/mL, dividing the patients into the HIGH (n=50) and LOW group (n=51) as outlined above. An overview of the cancer type by organs can be found in **Figure 4****.** The Patients in the HIGH group (n=50) were shown to remain on treatment for a longer time (24 weeks) relative to the LOW group (13 weeks) and had significantly better preliminary efficacy results **(Table 1).** Patients in the HIGH group seem to be stable and benefiting from the treatment for a longer period. Furthermore, there was no impact of exposure (i.e. between the LOW and HIGH Group) on safety, including severe adverse events (SAEs), related SAEs, severe AEs, or infusion-related reactions (IRRs).

### NSCLC-TR:

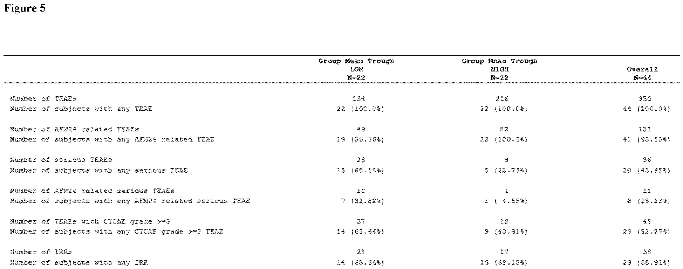

The median threshold for the NSCLC-TR Group was: 97641.5 ng/mL, dividing the patients into the HIGH (n=22) and LOW group (n=22) as outlined above. The Patients in the HIGH group were shown to remain on treatment for a longer time (32 weeks) relative to the LOW group (14 weeks) and had significantly better preliminary efficacy results **(Table 2),** which can also be seen in corresponding Kaplan-Meier Plots for PFS **(****Figure 1****).** Patients in the HIGH group seem to be stable and benefiting from the treatment for a longer period. Especially important is to mention, that the PFS curve show an early drop in the LOW group in the beginning (encircled), associated with an increased risk of early progression. Furthermore, there was no impact of exposure (i.e. between the LOW and HIGH Group) on safety, including SAEs, related SAEs, severe AEs, or IRRs (Figure 5).

### All-TR WITHOUT NSCLC-TR:

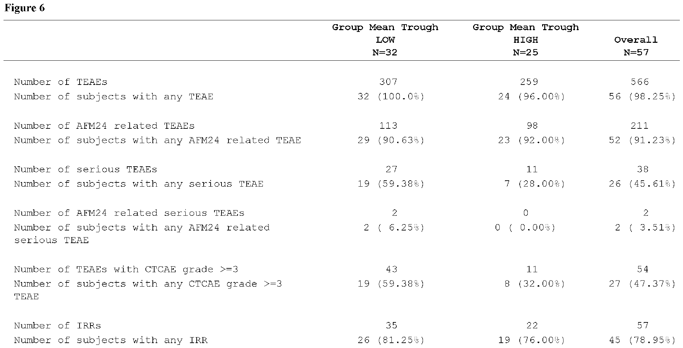

When removing the NSCLC subgroup patients from the All-TR Group, the corresponding PFS curve does not show the effect discussed above anymore **(****Figure 2****).** Considering safety, there was no impact of exposure (i.e. between the LOW and HIGH Group) on safety, including SAEs, related SAEs, severe AEs, or IRRs **(****Figure 6****).**

### AFM24-101 - PK Trough Values over time

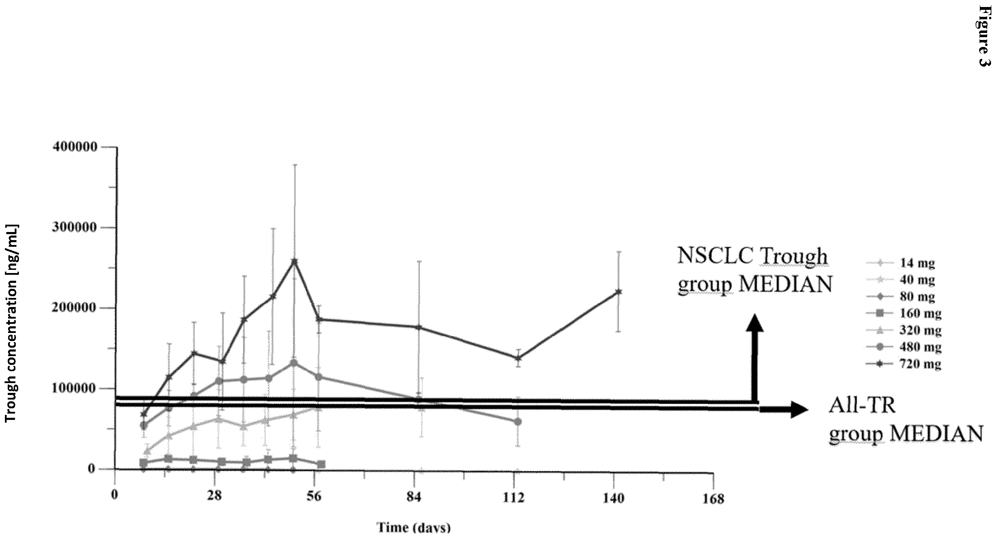

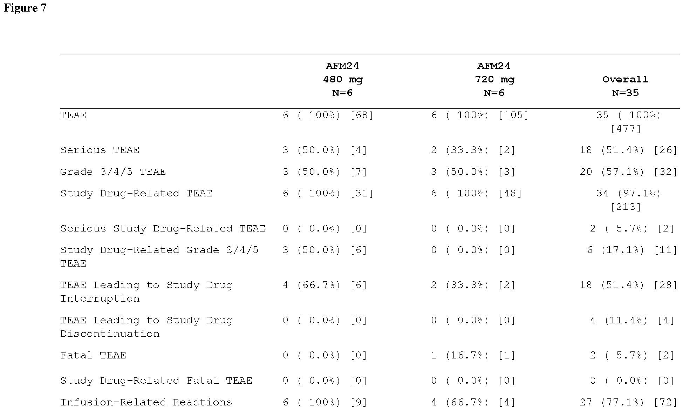

In order to set the data into perspective, the PK Trough Values over time for the different dosing cohorts of the AFM24-101 Phase 1 study and the All-TR Group MEDIAN and NSCLC-TR Group MEDIAN are shown in **Figure 3****.** Critically, the trough values over time in patients administered 720 mg qw in the AFM-101 study (N=6) correspond to exposure levels observed in the NSCLC-TR and in the All-TR HIGH exposure group **(****Figure 3****).** The 720 mg dose results in trough levels above the efficacy threshold in all patients by week 3. The safety of the 720 mg qw cohort in the AFM24-101 was comparable to the 480 mg cohort and the overall population of patients in this study **(****Figure 7****).**

### Overall Conclusions:

Patients in the High Groups do show significantly better efficacy. Based on the results presented, higher exposures to AFM24 have beneficial effect on patient response to treatment in particularly for patients with NSCLC, without affecting safety. Furthermore, to avoid the increased risk of early progression / early death in the LOW groups, a higher AFM24 dose to increase the trough levels seems beneficial.

### Tables:

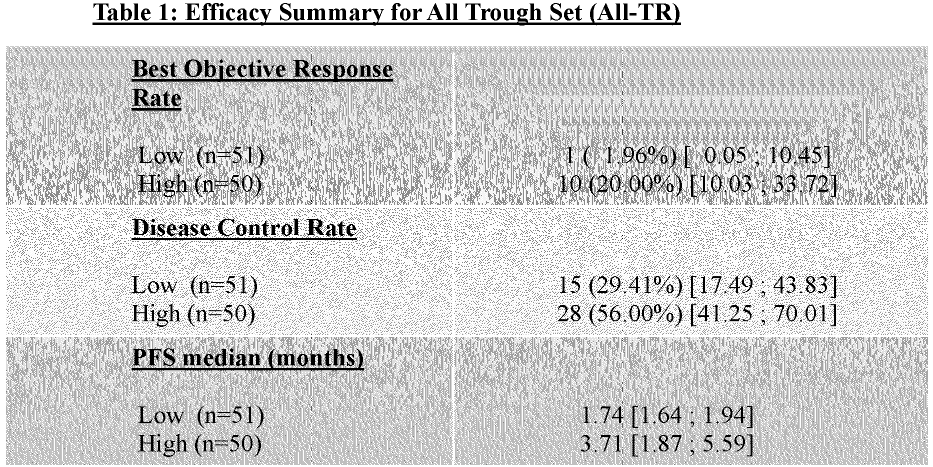

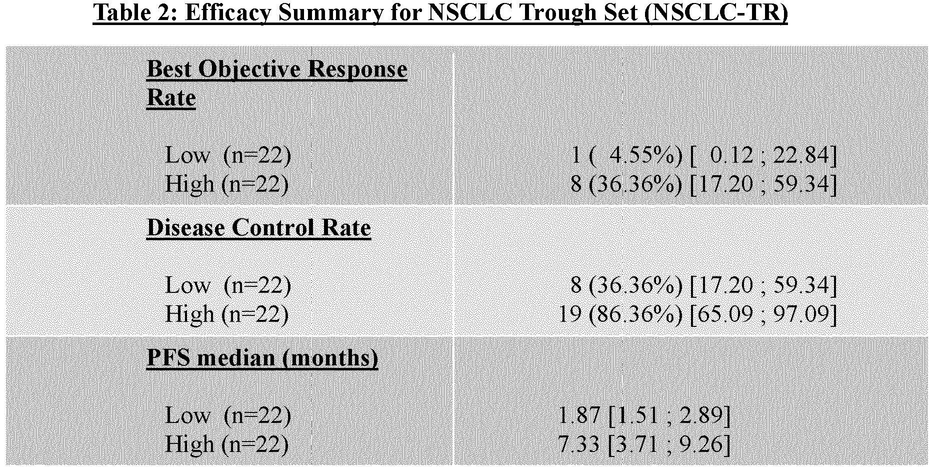

Embodiments illustratively described herein may suitably be practiced in the absence of any element or elements, limitation or limitations, not specifically disclosed herein. Thus, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present embodiments have been specifically disclosed by preferred embodiments and optional features, modification and variations thereof may be resorted to by those skilled in the art, and that such modifications and variations are considered to be within the scope of this invention. Each of the narrower species and subgeneric groupings falling within the generic disclosure also forms part of the invention. This includes the generic description of the invention with a proviso or negative limitation removing any subject matter from the genus, regardless of whether or not the excised material is specifically recited herein. In addition, where features are described in terms of Markush groups, those skilled in the art will recognize that the disclosure is also thereby described in terms of any individual member or subgroup of members of the Markush group.

Equivalents: Those skilled in the art will recognize or be able to ascertain using no more than routine experimentation, many equivalents to the specific embodiments of the invention described herein. Such equivalents are intended to be encompassed by the claims.

### Sequence Listing

| Seq ID NO | Description | Sequence |
|---|---|---|
| 1 | Linker L1 | GGSGGS |
| 2 | Linker L2 | GGS GGS GGS GGS GGS GGS |
| 3 | Linker L3 | GGSGGSGGSGGSGGSGGSGGS |
| 4 | Linker L4 | GGSGGSGGS |
| 5 | Linker L5 | GGGGS |
| 6 | Linker L6 | GGGGSGGGGS |
| 7 | Linker L7 | GGGGSGGGGSGGGGSGGGGS |
| 8 | Linker L8 | GGGGS GGGGS GGGGS GGGGS GGGGS GGGGS |
| 9 | CDR-H1 CD16A (LSIV21) | GYTFTSYY |
| 10 | CDR-H2 CD16A (LSIV21) | INPSGGST |
| 11 | CDR-H3 CD16A (LSIV21) | ARGSAYYYDFADY |
| 12 | CDR-L1 CD16A (LSIV21) | NIGSKN |
| | CDR-L2 CD16A (LSIV21) | QDN |
| 13 | CDR-L3 CD16A (LSIV21) | QVWDNYSVL |
| 14 | VH CD16A (LSIV21) | QVQLVQSGAEVKKPGESLKVSCKASGYTFTSYYMHWVRQAPGQGLEWMGIINPSGGSTSYA QKFQGRVTMTRDTSTSTVYMELSSLRSEDTAVYYCARGSAYYYDFADYWGQGTLVTVSS |
| 15 | VL CD16A (LSIV21) | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVL |
| 16 | scFv CD16A (LSIV21) | QVQLVQSGAEVKKPGESLKVSCKASGYTFTSYYMHWVRQAPGQGLEWMGIINPSGGSTSYA QKFQGRVTMTRDTSTSTVYMELSSLRSEDTAVYYCARGSAYYYDFADYWGQGTLVTVSSGG SGGSGGSGGSGGSGGSSYVLTQPSSVSVAPGQTATISCGGHNIGSKNVHWYQQRPGQSPVL VIYQDNKRPSGIPERFSGSNSGNTATLTISGTQAMDEADYYCQVWDNYSVLFGGGTKLTVL AAAGSHHHHHH |
| 17 | CDR-H1 CD16A (P2C-47) | GYTFTSYY |
| 18 | CDR-H2 CD16A (P2C-47) | IEPMYGST |
| 19 | CDR-H3 CD16A (P2C-47) | ARGSAYYYDFADY |
| 20 | CDR-L1 CD16A (P2C-47) | NIGSKN |
| | CDR-L2 CD16A (P2C-47) | QDN |
| 21 | CDR-L3 CD16A (P2C-47) | QVWDNYSVL |
| 22 | VH CD16A (P2C-47) | QVQLVQSGAEVKKPGESLKVSCKASGYTFTSYYMHWVRQAPGQGLEWMGAIEPMYGSTSYA QKFQGRVTMTRDTSTSTVYMELSSLRSEDTAVYYCARGSAYYYDFADYWGQGTLVTVSS |
| 23 | VL CD16A (P2C-47) | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVL |
| 24 | scFv CD16A (P2C-47) | QVQLVQSGAEVKKPGESLKVSCKASGYTFTSYYMHWVRQAPGQGLEWMGAIEPMYGSTSYA QKFQGRVTMTRDTSTSTVYMELSSLRSEDTAVYYCARGSAYYYDFADYWGQGTLVTVSSGG SGGSGGSGGSGGSGGSSYVLTQPSSVSVAPGQTATISCGGHNIGSKNVHWYQQRPGQSPVL VIYQDNKRPSGIPERFSGSNSGNTATLTISGTQAMDEADYYCQVWDNYSVLFGGGTKLTVL AAAGSHHHHHH |
| 25 | CDR-H1 CD16A (ABC1197) | GYTFTNYY |
| 26 | CDR-H2 CD16A (ABC1197) | INPSGGVT |
| 27 | CDR-H3 CD16A (ABC1197) | ARGSAYYYDFADY |
| 28 | CDR-L1 CD16A (ABC1197) | NIGSKS |
| | CDR-L2 CD16A (ABC1197) | QDK |
| 29 | CDR-L3 CD16A (ABC1197) | QVWDDYIVL |
| 30 | VH CD16A (ABC1197) | QVQLVQSGAEVKKPGESLKVSCKASGYTFTNYYMQWVRQAPGQGLEWMGIINPSGGVTSYA QKFQGRVTMTRDTSTSTVYMELSSLRSEDTAVYYCARGSAYYYDFADYWGQGTLVTVSS |
| 31 | VL CD16A (ABC1197) | SYVLTQPSSVSVAPGQTARITCGGNNIGSKSVHWYQQKPGQAPVLVIYQDKKRPSGIPERF SGSNSGNTATLTISGTQAMDEADYYCQVWDDYIVLFGGGTKLTVL |
| 32 | CDR-H1 CD16A (P2C-47var) | SYYMH |
| 33 | CDR-H2 CD16A (P2C-47var) | AIEPRYGSTSYAQKFQG |
| 34 | CDR-H3 CD16A (P2C-47var) | GSAYYYDFADY |
| 35 | CDR-L1 CD16A (P2C-47var) | GGHNIGSKNVH |
| 36 | CDR-L2 CD16A (P2C-47var) | QDNKRPS |
| 37 | CDR-L3 CD16A (P2C-47var) | QVWDNYNVL |
| 38 | CDR-H1 CD16A (ABC1197var) | NYYMQ |
| 39 | CDR-H2 CD16A (ABC1197var) | IINPSGGVTSYAQKFQG |
| 40 | CDR-H3 CD16A (ABC1197var) | GSAYYYDFADY |
| 41 | CDR-L1 CD16A (ABC1197var) | GGNNIGSKSVH |
| 42 | CDR-L2 CD16A (ABC1197var) | QDKKRPS |
| 43 | CDR-L3 CD16A (ABC1197var) | QVWDDYIVL |
| 44 | CD16A (P2C47var46) VH | EVQLVQSGAEVKKPGASVKVSCKASGYTFTSYYMHWVRQAPGQCLEWMGAIEPTYGSTSYA QKFQGRVTMTRDTSTSTVYMELSSLRSEDTAVYYCARGSAYYYDFADYWGQGTLVTVSS |
| 45 | CD16A (P2C47var46) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 46 | CD16A P2C47var50 VH | QVQLVQSGAEVKKPGASVKVSCKASGYTFTSYYMHWVRQAPGQCLEWMGAIEPTYGSTSYA QKFQGRVTMTRDTSTSTVYMELSSLRSEDTAVYYCARGSAYYYDFADYWGQGTLVTVSS |
| 47 | CD16A (P2C47var50) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 48 | CD16A P2C47var51 VH | QVQLVQSGAEVKKPGASVKVSCKASGYTFTSYYMHWVRQAPGQCLEWMGAIEPRYGSTSYA QKFQGRVTMTRDTSTSTVYMELSSLRSEDTAVYYCARGSAYYYDFADYWGQGTLVTVSS |
| 49 | CD16A (P2C47var51) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 50 | VL CD16A (P2C47var52) VH | QVQLVQSGAEVKKPGASVKVSCKASGYTFTSYYMHWVRQAPGQCLEWMGAIEPTYGSTSYA QKFQGRVTMTRDTSTSTVYMELSSLRSEDTAVYYCARGSAYYYDFADYWGQGTLVTVS |
| 51 | CD16A (P2C47var52) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 52 | CD16A (ABC1197var8 ) VH | QVQLVQSGAEVKKPGASVKVSCKASGYTFTNYYMQWVRQAPGQCLEWMGIINPSGGVTSYA QKFQGRVTMTRDTSTSTVYMELSSLRSEDTAVYYCARGSAYYYDFADYWGQGTLVTVSS |
| 53 | CD16A (ABC1197var8 ) VL | SYELTQPLSVSVALGQTARITCGGNNIGSKSVHWYQQKPGQAPVLVIYQDKKRPSGIPERF SGSNSGNTATLTISRAQAGDEADYYCQVWDDYIVLFGCGTKLTVL |
| 54 | CD16A ABC1197var11 VH | QVQLVQSGAEVKKPGASVKVSCKASGYTFTNYYMQWVRQAPGQCLEWMGIINPSGGVTSYA QKFQGRVTMTRDTSTSTVYMELSSLRSEDTAVYYCARGSAYYYDFADYWGQGTLVTVSSG |
| 55 | CD16A (ABC1197var8 ) VL | SYELTQPLSVSVALGQTARITCGGNNIGSKSVHWYQQKPGQAPVLVIYQDKKRPSGIPERF SGSNSGNTATLTISRAQAGDEADYYCQVWDDYIVLFGCGTKLTVL |
| 56 | CDR-H1 EGFR | GGSVSSGSYY |
| 57 | CDR-H2 EGFR | IYYSGST |
| 58 | CDR-H3 EGFR | ARNPISIPAFDI |
| 59 | CDR-L1 EGFR | NIGSKS |
| | CDR-L2 EGFR | YDS |
| 60 | CDR-L3 EGFR | QVWDTSSDHVL |
| 61 | VH EGFR | QVQLQESGPGLVKPSETLSLTCTVSGGSVSSGSYYWSWIRQPPGKGLEWIGYIYYSGSTNY NPSLKSRVTISVDTSKNQFSLKLSSVTAADTAVYYCARNPISIPAFDIWGQGTMVTVSS |
| 62 | VL EGFR | QPVLTQPPSVSVAPGKTARITCGGNNIGSKSVHWYQQKPGQAPVLVIYYDSDRPSGIPERF SGSNSGNTATLTISRVEAGDEADYYCQVWDTSSDHVLFGGGTKLTVL |
| 63 | AFM24 H-chain | QVQLVQSGAEVKKPGESLKVSCKASGYTFTSYYMHWVRQAPGQGLEWMGIINPSGGSTSYA QKFQGRVTMTRDTSTSTVYMELSSLRSEDTAVYYCARGSAYYYDFADYWGQGTLVTVSSAS TKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEFEGGPSVF LFPPKPKDTLMISRTPEVTCVVVAVSHEDPEVKFNWYVDGVEVHNAKTKPREEQYNSTYRV VSVLTVLHQDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRWQQGNVFS CSVMHEALHNHYTQKSLSLSPGGGGGSGGGGSQVQLQESGPGLVKPSETLSLTCTVSGGSV SSGSYYWSWIRQPPGKGLEWIGYIYYSGSTNYNPSLKSRVTISVDTSKNQFSLKLSSVTAA DTAVYYCARNPISIPAFDIWGQGTMVTVSSGGSGGSGGSGGSGGSGGSQPVLTQPPSVSVA PGKTARITCGGNNIGSKSVHWYQQKPGQAPVLVIYYDSDRPSGIPERFSGSNSGNTATLTI SRVEAGDEADYYCQVWDTSSDHVLFGGGTKLTVL |
| 64 | AFM24 L-chain | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVLGQPKAAPSVTLFPPSS EELQANKATLVCLISDFYPGAVTVAWKADSSPVKAGVETTTPSKQSNNKYAASSYLSLTPE QWKSHRSYSCQVTHEGSTVEKTVAPTECS |
| 65 | AFM24-2 H-chain | QVQLVQSGAEVKKPGESLKVSCKASGYTFTSYYMHWVRQAPGQGLEWMGAIEPMYGSTSYA QKFQGRVTMTRDTSTSTVYMELSSLRSEDTAVYYCARGSAYYYDFADYWGQGTLVTVSSAS TKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEFEGGPSVF LFPPKPKDTLMISRTPEVTCVVVAVSHEDPEVKFNWYVDGVEVHNAKTKPREEQYNSTYRV VSVLTVLHQDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRWQQGNVFS CSVMHEALHNHYTQKSLSLSPGGGGGSGGGGSQVQLQESGPGLVKPSETLSLTCTVSGGSV SSGSYYWSWIRQPPGKGLEWIGYIYYSGSTNYNPSLKSRVTISVDTSKNQFSLKLSSVTAA DTAVYYCARNPISIPAFDIWGQGTMVTVSSGGSGGSGGSGGSGGSGGSQPVLTQPPSVSVA PGKTARITCGGNNIGSKSVHWYQQKPGQAPVLVIYYDSDRPSGIPERFSGSNSGNTATLTI SRVEAGDEADYYCQVWDTSSDHVLFGGGTKLTVL |
| 66 | AFM24-2 L-chain | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVLGQPKAAPSVTLFPPSS EELQANKATLVCLISDFYPGAVTVAWKADSSPVKAGVETTTPSKQSNNKYAASSYLSLTPE QWKSHRSYSCQVTHEGSTVEKTVAPTECS |
| 67 | AFM24_T | QVQLQESGPGLVKPSETLSLTCTVSGGSVSSGSYYWSWIRQPPGKGLEWIGYIYYSGSTNY NPSLKSRVTISVDTSKNQFSLKLSSVTAADTAVYYCARNPISIPAFDIWGQGTMVTVSSGG SGGSGGSSYVLTQPSSVSVAPGQTATISCGGHNIGSKNVHWYQQRPGQSPVLVIYQDNKRP SGIPERFSGSNSGNTATLTISGTQAMDEADYYCQVWDNYSVLFGGGTKLTVLGGSGGSQVQ LVQSGAEVKKPGESLKVSCKASGYTFTSYYMHWVRQAPGQGLEWMGIINPSGGSTSYAQKF QGRVTMTRDTSTSTVYMELSSLRSEDTAVYYCARGSAYYYDFADYWGQGTLVTVSSGGSGG SGGSQPVLTQPPSVSVAPGKTARITCGGNNIGSKSVHWYQQKPGQAPVLVIYYDSDRPSGI PERFSGSNSGNTATLTISRVEAGDEADYYCQVWDTSSDHVLFGGGTKLTVLAAAGSHHHHH H |
| 68 | AFM24_Bi-scFv-Fc | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVLGQPKAAPSVTLFPPSS EELQANKATLVCLISDFYPGAVTVAWKADSSPVKAGVETTTPSKQSNNKYAASSYLSLTPE QWKSHRSYSCQVTHEGSTVEKTVAPTECSLTVDKSRWQQGNVFSCSVMHEALHNHYTQKSL SLSPGGGGGSGGGGSQVQLQESGPGLVKPSETLSLTCTVSGGSVSSGSYYWSWIRQPPGKG LEWIGYIYYSGSTNYNPSLKSRVTISVDTSKNQFSLKLSSVTAADTAVYYCARNPISIPAF DIWGQGTMVTVSSGGSGGSGGSGGSGGSGGSQPVLTQPPSVSVAPGKTARITCGGNNIGSK SVHWYQQKPGQAPVLVIYYDSDRPSGIPERFSGSNSGNTATLTISRVEAGDEADYYCQVWD TSSDHVLFGGGTKLTVL |
| 69 | Hinge - CH2 domain - CH3 domain | EPKSCDKTHTCPPCPAPEFEGGPSVFLFPPKPKDTLMISRTPEVTCVVVAVSHEDPEVKFN WYVDGVEVHNAKTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQPENNYKTTPPVL DSDGSFFLYSKLTVDKSRWQQGNVFSCSVMHEALHNHYTQKSLSLSPG |
| 70 | C-terminal epitope of CD16A | SFFPPGYQ |
| 71 | human CD16A | MWQLLLPTALLLLVSAGMRTEDLPKAVVFLEPQWYRVLEKDSVTLKCQGAYSPEDNSTQWF HNESLISSQASSYFIDAATVDDSGEYRCQTNLSTLSDPVQLEVHIGWLLLQAPRWVFKEED PIHLRCHSWKNTALHKVTYLQNGKGRKYFHHNSDFYIPKATLKDSGSYFCRGLFGSKNVSS ETVNITITQGLAVSTISSFFPPGYQVSFCLVMVLLFAVDTGLYFSVKTNIRSSTRDWKDHK FKWRKDPQDK |
| 72 | Linker | GGGS |
| 73 | Hexahistidin e tag | HHHHHH |

## Claims

1. A bispecific antibody construct for use in a method of treating non-small cell lung cancer (NSCLC) in a subject, wherein the bispecific antibody construct comprises at least one first binding domain capable of specifically binding CD16A and at least one second binding domain capable of specifically binding EGFR, wherein the method comprises administering the bispecific antibody construct in a loading dose for a first period of time, and administering the bispecific antibody construct in a maintenance dose for a second period of time, wherein the loading dose is higher than the maintenance dose.

2. The bispecific antibody construct for the use of claim 1, wherein the method further comprises administering to the subject an immune checkpoint inhibitor.

3. The bispecific antibody construct for the use of claim 1 or 2, wherein the tumor **characterized by** epidermal growth factor receptor (EGFR) overexpression on tumor cells, optionally wherein the tumor is an EGFR-positive tumor.

4. The bispecific antibody construct for the use of any one of the preceding claims, wherein the first binding domain binds to an epitope on CD16A which is C-terminal to the physiological Fcy receptor binding domain, said epitope preferably comprising Y158 of SEQ ID NO: 1.

5. The bispecific antibody construct for the use of any one of the preceding claims, wherein the first binding domain comprises
(i) a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13);
(ii) a VH region comprising CDR-H1 sequence GYTFTSYY (SEQ ID NO: 17), CDR-H2 sequence IEPMYGST (SEQ ID NO: 18), and CDR-H3 sequence ARGSAYYYDFADY (SEQ ID NO: 19), and a VL region comprising CDR-L1 sequence NIGSKN (SEQ ID NO: 20), CDR-L2 sequence QDN, and CDR-L3 sequence QVWDNYSVL (SEQ ID NO: 21);
(iii) a VH region comprising CDR-H1 sequence GYTFTNYY (SEQ ID NO: 25), CDR-H2 sequence INPSGGVT (SEQ ID NO: 26), and CDR-H3 sequence ARGSAYYYDFADY (SEQ ID NO: 27), and a VL region comprising CDR-L1 sequence NIGSKS (SEQ ID NO: 28), CDR-L2 sequence QDK, and CDR-L3 sequence QVWDDYIVL (SEQ ID NO: 29);
(iv) a VH region comprising CDR-H1 sequence SYYMH (SEQ ID NO: 32), CDR-H2 sequence AIEPRYGSTSYAQKFQG (SEQ ID NO: 33), and CDR-H3 sequence GSAYYYDFADY (SEQ ID NO: 34), and a VL region comprising CDR-L1 sequence GGHNIGSKNVH (SEQ ID NO: 35), CDR-L2 sequence QDNKRPS (SEQ ID NO: 36), and CDR-L3 sequence QVWDNYNVL (SEQ ID NO: 37); or
(v) a VH region comprising CDR-H1 sequence NYYMQ (SEQ ID NO: 38), CDR-H2 sequence IINPSGGVTSYAQKFQG (SEQ ID NO: 39), and CDR-H3 sequence GSAYYYDFADY (SEQ ID NO: 40), and a VL region comprising CDR-L1 sequence GGNNIGSKSVH (SEQ ID NO: 41), CDR-L2 sequence QDKKRPS (SEQ ID NO: 42), and a CDR-L3 sequence QVWDDYIVL (SEQ ID NO: 43);
or wherein the first binding domain comprises a
(i) a VH region having at least 80%, at least 85%, at least 90%, at least 95%, at least 98%, or at least 99% sequence identity to SEQ ID NO: 14 and a VL region having at least 80%, at least 85%, at least 90%, at least 95%, at least 98%, or at least 99% sequence identity to SEQ ID NO: 15;
(ii) a VH region having at least 80%, at least 85%, at least 90%, at least 95%, at least 98%, or at least 99% sequence identity to SEQ ID NO: 22 and a VL region having at least 80%, at least 85%, at least 90%, at least 95%, at least 98%, or at least 99% sequence identity to SEQ ID NO: 23;
(iii) a VH region having at least 80%, at least 85%, at least 90%, at least 95%, at least 98%, or at least 99% sequence identity to SEQ ID NO: 30 and a VL region having at least 80%, at least 85%, at least 90%, at least 95%, at least 98%, or at least 99% sequence identity to SEQ ID NO: 31;
(iv) a VH region having at least 80%, at least 85%, at least 90%, at least 95%, at least 98%, or at least 99% sequence identity to SEQ ID NO: 44 and a VL region having at least 80%, at least 85%, at least 90%, at least 95%, at least 98%, or at least 99% sequence identity to SEQ ID NO: 45;
(v) a VH region having at least 80%, at least 85%, at least 90%, at least 95%, at least 98%, or at least 99% sequence identity to SEQ ID NO: 46 and a VL region having at least 80%, at least 85%, at least 90%, at least 95%, at least 98%, or at least 99% sequence identity to SEQ ID NO: 47;
(vi) a VH region having at least 80%, at least 85%, at least 90%, at least 95%, at least 98%, or at least 99% sequence identity to SEQ ID NO: 48 and a VL region having at least 80%, at least 85%, at least 90%, at least 95%, at least 98%, or at least 99% sequence identity to SEQ ID NO: 49;
(vii) a VH region having at least 80%, at least 85%, at least 90%, at least 95%, at least 98%, or at least 99% sequence identity to SEQ ID NO: 50 and a VL region having at least 80%, at least 85%, at least 90%, at least 95%, at least 98%, or at least 99% sequence identity to SEQ ID NO: 51;
(viii) a VH region having at least 80%, at least 85%, at least 90%, at least 95%, at least 98%, or at least 99% sequence identity to SEQ ID NO: 52 and a VL region having at least 80%, at least 85%, at least 90%, at least 95%, at least 98%, or at least 99% sequence identity to SEQ ID NO: 53; or
(ix) a VH region having at least 80%, at least 85%, at least 90%, at least 95%, at least 98%, or at least 99% sequence identity to SEQ ID NO: 54 and a VL region having at least 80%, at least 85%, at least 90%, at least 95%, at least 98%, or at least 99% sequence identity to SEQ ID NO: 55;
or wherein the first binding domain comprises a
(i) a VH region as depicted in SEQ ID NO: 14 and a VL region as depicted in SEQ ID NO: 15;
(ii) a VH region as depicted in SEQ ID NO: 22 and a VL region as depicted in SEQ ID NO: 23;
(iii) a VH region as depicted in SEQ ID NO: 30 and a VL region as depicted in SEQ ID NO: 31;
(iv) a VH region as depicted in SEQ ID NO: 44 and a VL region as depicted in SEQ ID NO: 45;
(v) a VH region as depicted in SEQ ID NO: 46 and a VL region as depicted in SEQ ID NO: 47;
(vi) a VH region as depicted in SEQ ID NO: 48 and a VL region as depicted in SEQ ID NO: 49;
(vii) a VH region as depicted in SEQ ID NO: 53 and a VL region as depicted in SEQ ID NO: 50;
(viii) a VH region as depicted in SEQ ID NO: 55 and a VL region as depicted in SEQ ID NO: 51; or
(ix) a VH region as depicted in SEQ ID NO: 52 and a VL region as depicted in SEQ ID NO: 53;
optionally wherein the first binding domain is a variable domain (Fv), a single chain Fv (scFv), a Fab, a single chain diabody (scDb), a diabody (Db) or a double Fab.

6. The bispecific antibody construct for the use of any one of the preceding claims, wherein the second binding domain comprises a VH and a VL domain of an antibody; optionally
wherein the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60), or
wherein the second binding domain comprises a VH region having at least 80%, at least 85%, at least 90%, at least 95%, at least 98%, or at least 99% sequence identity to SEQ ID NO: 61 and a VL region having at least 80%, at least 85%, at least 90%, at least 95%, at least 98%, or at least 99% sequence identity to SEQ ID NO: 62, preferably
wherein the second binding domain comprises a VH region as depicted in SEQ ID NO: 61 and a VL region as depicted in SEQ ID NO: 62,
wherein the second binding domain is optionally a variable domain (Fv), a single chain Fv (scFv), a Fab, a single chain diabody (scDb), a diabody (Db) or a double Fab, optionally wherein the bispecific antibody construct binds to a target cell and an immune effector cell simultaneously,
optionally wherein the bispecific antibody construct is AFM24.

7. The bispecific antibody construct for the use of any one of the preceding claims, wherein the immune checkpoint inhibitor is a PD-L1 or PD-1 inhibitor, optionally wherein the immune checkpoint inhibitor is an anti-PD-1 antibody or an anti-PD-L1 antibody, optionally wherein the immune checkpoint inhibitor is selected from the group consisting of nivolumab, atezolizumab, pembrolizumab cemiplimab, H4H7798N, dostarlimab, durvalumab, pidilizumab, MED10608, BI 754091, PF-0680159, spartalizumab, camrelizumab, JNJ-63723283, MCLA-134, H2M8314N, avelumab, MDX-1105, LY3300054, FAZ053, STI-1014, CX-072, KN035, and CK-301, preferably wherein the immune checkpoint inhibitor is selected from the group consisting of nivolumab atezolizumab and pembrolizumab, most preferably wherein the immune checkpoint inhibitor is atezolizumab.

8. The bispecific antibody construct for the use of any one of the preceding claims, wherein the bispecific antibody construct is administered to the subject once every about 6 to about 8 days, or once every about 7 days, and/or
wherein the loading dose of the bispecific antibody construct is administered at a dose in the range of about 600 to about 1200 mg, about 600 to about 850 mg, about 650 to about 800 mg, about 700 to about 750 mg, or about 720 mg, and/or
wherein the maintenance dose of the bispecific antibody construct is administered at a dose in the range of about 300 to about 550 mg, about 350 to about 550 mg, about 400 to about 550 mg, about 420 to about 530 mg, about 450 to about 500 mg, or about 480 mg.

9. The bispecific antibody construct for the use of any one of the preceding claims, wherein a cycle of administering consists of administering the bispecific antibody construct molecule 4 times, and optionally administering the immune checkpoint inhibitor 2 time, and treatment of the subject is carried out for at least one cycle, and/or wherein a cycle of administering is about 4 weeks, and/or
wherein the loading dose of the bispecific antibody is administered for up to about 4 cycles, up to about 3 cycles, about to about 2 cycles, about 1 to about 3 cycles, about 1 to about 2 cycles, or about 2 cycles, and/or
wherein the loading dose of the bispecific antibody is administered for up to about 16 weeks, up to about 12 weeks, about to about 8 weeks, about 4 to about 12 weeks, about 4 to about 10 weeks, about 6 to about 10 weeks, about 9 to about 11 weeks, or about 8 weeks.

10. The bispecific antibody construct for the use of any one of the preceding claims, wherein the immune checkpoint inhibitor is administered to the subject once every about 1 to about 3 weeks, once every about 2 weeks, once every about 12 to about 16 days, once every about 13 to about 15 days, or once every about 14 days, and/or wherein the immune checkpoint inhibitor is administered at a dose in the range of about 500 to about 1200 mg, about 700 to about 1000 mg, about 800 to about 900 mg, or about 840 mg, and/or
wherein the immune checkpoint inhibitor is administered in the first and the third week of each cycle, and/or
wherein a dose the immune checkpoint inhibitor and a dose of the bispecific antibody construct are administered to the subject at the same time, or about the same time, and/or wherein a dose the immune checkpoint inhibitor is concurrently administered with a dose of the bispecific antibody construct, and/or
wherein a dose of the bispecific antibody molecule and/or a dose the immune checkpoint inhibitor are administered as split-day dosing on two consecutive days.

11. The bispecific antibody construct for the use of any one of the preceding claims, wherein a lead-in dose of the bispecific antibody construct is administered to the subject about 5 to about 8 days or about 6 to about 7 days prior to a first cycle of administration, optionally wherein the lead-in dose is about the same dose as the loading dose, optionally wherein the lead-in dose is in the range of about 600 to about 850 mg, about 650 to about 800 mg, about 700 to about 750 mg, or about 720 mg, optionally wherein the lead-in dose is administered as a split-dose on two consecutive days.

12. The bispecific antibody construct for the use of any one of the preceding claims, wherein the median trough plasma concentration of the bispecific antibody construct is about 97000 ng/mL, and/or
wherein the expected median progression free survival of the subject or a group of subjects suffering from NSCLC is at least about 3 months, at least about 3.5 months, at least about 4 months, at least about 4.5 months, at least about 5 months, at least about 5.5 months, at least about 6 months, at least about 6.5 months, or at least about 7 months after begin of the treatment, and/or
wherein the use comprises prior to treatment stratifying the patient based on the type of the tumor and selecting the patient for treatment as defined in any one of claims 1-11 after the patient was diagnosed with NSCLC.

13. A composition comprising an immune checkpoint inhibitor and a bispecific antibody construct that comprises at least one first binding domain capable of specifically binding CD16A and at least one second binding domain capable of specifically binding EGFR, wherein the immune checkpoint inhibitor is present in an amount (by weight) that is in the range of about 1.0 to about 1.3 times, about 1.1 to about 1.2 times, or about 1.15 to about 1.18 times the amount of the bispecific antibody construct, optionally for use in a method as defined in any one of claims 1-12.

14. A bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of treating NSCLC in a subject and providing an expected median progression free survival of the subject or a group of subjects suffering from NSCLC that is about at least about 3 months, at least about 3.5 months, at least about 4 months, at least about 4.5 months, at least about 5 months, at least about 5.5 months, at least about 6 months, at least about 6.5 months, or at least about 7 months after begin of the treatment, optionally wherein the use is in a method as defined in any one of claims 1-12.

15. An immune checkpoint inhibitor of a combination of an immune checkpoint inhibitor and a bispecific antibody construct, for use in a method of treating non-small cell lung cancer (NSCLC) in a subject, wherein the bispecific antibody construct comprises at least one first binding domain capable of specifically binding CD16A and at least one second binding domain capable of specifically binding EGFR, wherein the method comprises administering the bispecific antibody construct in a loading dose for a first period of time, and administering the bispecific antibody construct in a maintenance dose for a second period of time, wherein the loading dose is higher than the maintenance dose, wherein the method is as defined in any one of claims 1-12.
